# EUROPEAN PATENT APPLICATION

(11) **EP 1 327 638 A1**
(43) Date of publication of application: **16.07.2003**
(21) Application number: 01965588.5
(22) Date of filing: 12.09.2001
(51) Int. Cl.: C07K 14/47, C12N 15/12, C07K 16/18, C12N 5/08, G01N 33/53

(54) **NOVEL DENDRITIC CELL WALL MEMBRANE AND USE THEREOF**

(30) Priority: 12.09.2000 JP 2000277352
(71) Applicant: KIRIN BEER KABUSHIKI KAISHA, Tokyo 104-8288 (JP)
(72) Inventor: WATARAI, Hiroshi, c/o Kirin Beer Kabushiki Kaisha, Takasaki-shi, Gunma 370-1295 (JP); YAMAGUCHI, Yasunori, c/o Kirin Beer Kabushiki K., Takasaki-shi, Gunma 370-1295 (JP); HINOHARA, Atsushi, c/o Kirin Beer Kabushiki Kaisha, Takasaki-shi, Gunma 370-1295 (JP); NAKAGAWA, Ryusuke, c/o Kirin Beer Kahushiki Kaisha, Takasaki-shi, Gunma 370-1295 (JP); EHARA, Hiromi, c/o Kirin Beer Kabushiki Kaisha, Takasaki-shi, Gunma 370-1295 (JP); IMAI, Naoshi, c/o Kirin Beer Kabushiki Kaisha, Takasaki-shi, Gunma 370-1295 (JP)
(74) Representative: HOFFMANN - EITLE
(86) International application number: JP0107919
(87) International publication number: WO02022683

(57) **Abstract**

The present invention relates to an isolated human dendritic cell membrane molecule having the amino acid sequence represented by SEQ ID NO: 1 which shows a significant increase in expression with the maturation of human dendritic cells (DC), a variant thereof, a DNA encoding the same, and a method for separating or detecting dendritic cells using the membrane molecule or its variant.

SEQUENCE LISTING

<110> Kirin Beer Kabushiki Kaisha

<120> New Dendritic Cell Membrane Molecule and Its Uses

<130> PH-1297-PCT

<140>
<141>

<150> JP 2000-277352
<151> 2000-09-12

<160> 8

<170> PatentIn Ver. 2.0

<210> 1
<211> 534
<212> PRT
<213> Homo sapiens

<400> 1
Met Leu Arg Arg Arg Gly Ser Pro Gly Met Gly Val His Val Gly Ala
1               5                   10                  15

Ala Leu Gly Ala Leu Trp Phe Cys Leu Thr Gly Ala Leu Glu Val Gln
            20                  25                  30

Val Pro Glu Asp Pro Val Val Ala Leu Val Gly Thr Asp Ala Thr Leu
        35                  40                  45
Cys Cys Ser Phe Ser Pro Glu Pro Gly Phe Ser Leu Ala Gln Leu Asn
    50                  55                  60
Leu Ile Trp Gln Leu Thr Asp Thr Lys Gln Leu Val His Ser Phe Ala
65                  70                  75                  80
Glu Gly Gln Asp Gln Gly Ser Ala Tyr Ala Asn Arg Thr Ala Leu Phe
                85                  90                  95
Pro Asp Leu Leu Ala Gln Gly Asn Ala Ser Leu Arg Leu Gln Arg Val
            100                 105                 110
Arg Val Ala Asp Glu Gly Ser Phe Thr Cys Phe Val Ser Ile Arg Asp
        115                 120                 125
Phe Gly Ser Ala Ala Val Ser Leu Gln Val Ala Ala Pro Tyr Ser Lys
    130                 135                 140
Pro Ser Met Thr Leu Glu Pro Asn Lys Asp Leu Arg Pro Gly Asp Thr
145                 150                 155                 160
Val Thr Ile Thr Cys Ser Ser Tyr Gln Gly Tyr Pro Glu Ala Glu Val
                165                 170                 175
Phe Trp Gln Asp Gly Gln Gly Val Pro Leu Thr Gly Asn Val Thr Thr
            180                 185                 190

Ser Gln Met Ala Asn Glu Gln Gly Leu Phe Asp Val His Ser Ile Leu
        195                 200                 205
Arg Val Val Leu Gly Ala Asn Gly Thr Tyr Ser Cys Leu Val Arg Asn
    210                 215                 220
Pro Val Leu Gln Gln Asp Ala His Ser Ser Val Thr Ile Thr Pro Gln
225                 230                 235                 240
Arg Ser Pro Thr Gly Ala Val Glu Val Gln Val Pro Glu Asp Pro Val
                245                 250                 255
Val Ala Leu Val Gly Thr Asp Ala Thr Leu Arg Cys Ser Phe Ser Pro
            260                 265                 270
Glu Pro Gly Phe Ser Leu Ala Gln Leu Asn Leu Ile Trp Gln Leu Thr
        275                 280                 285
Asp Thr Lys Gln Leu Val His Ser Phe Thr Glu Gly Arg Asp Gln Gly
    290                 295                 300
Ser Ala Tyr Ala Asn Arg Thr Ala Leu Phe Pro Asp Leu Leu Ala Gln
305                 310                 315                 320
Gly Asn Ala Ser Leu Arg Leu Gln Arg Val Arg Val Ala Asp Glu Gly
                325                 330                 335
Ser Phe Thr Cys Phe Val Ser Ile Arg Asp Phe Gly Ser Ala Ala Val
            340                 345                 350

Ser Leu Gln Val Ala Ala Pro Tyr Ser Lys Pro Ser Met Thr Leu Glu
        355                 360                 365
Pro Asn Lys Asp Leu Arg Pro Gly Asp Thr Val Thr Ile Thr Cys Ser
    370                 375                 380
Ser Tyr Arg Gly Tyr Pro Glu Ala Glu Val Phe Trp Gln Asp Gly Gln
385                 390                 395                 400
Gly Val Pro Leu Thr Gly Asn Val Thr Thr Ser Gln Met Ala Asn Glu
                405                 410                 415
Gln Gly Leu Phe Asp Val His Ser Val Leu Arg Val Val Leu Gly Ala
            420                 425                 430
Asn Gly Thr Tyr Ser Cys Leu Val Arg Asn Pro Val Leu Gln Gln Asp
        435                 440                 445
Ala His Gly Ser Val Thr Ile Thr Gly Gln Pro Met Thr Phe Pro Pro
    450                 455                 460
Glu Ala Leu Trp Val Thr Val Gly Leu Ser Val Cys Leu Ile Ala Leu
465                 470                 475                 480
Leu Val Ala Leu Ala Phe Val Cys Trp Arg Lys Ile Lys Gln Ser Cys
                485                 490                 495
Glu Glu Glu Asn Ala Gly Ala Glu Asp Gln Asp Gly Glu Gly Glu Gly
            500                 505                 510

Ser Lys Thr Ala Leu Gln Pro Leu Lys His Ser Asp Ser Lys Glu Asp
        515                 520                 525
Asp Gly Gln Glu Ile Ala
    530
<210> 2
<211> 1605
<212> DNA
<213> Homo sapiens
<400> 2
atgctgcgtc ggcggggcag ccctggcatg ggtgtgcatg tgggtgcagc cctgggagca 60
ctgtggttct gcctcacagg agccctggag gtccaggtcc ctgaagaccc agtggtggca 120
ctggtgggca ccgatgccac cctgtgctgc tccttctccc ctgagcctgg cttcagcctg 180
gcacagctca acctcatctg gcagctgaca gataccaaac agctggtgca cagctttgct 240
gagggccagg accagggcag cgcctatgcc aaccgcacgg ccctcttccc ggacctgctg 300
gcacagggca acgcatccct gaggctgcag cgcgtgcgtg tggcggacga gggcagcttc 360
acctgcttcg tgagcatccg ggatttcggc agcgctgccg tcagcctgca ggtggccgct 420
ccctactcga agcccagcat gaccctggag cccaacaagg acctgcggcc aggggacacg 480
gtgaccatca cgtgctccag ctaccagggc taccctgagg ctgaggtgtt ctggcaggat 540
gggcagggtg tgcccctgac tggcaacgtg accacgtcgc agatggccaa cgagcagggc 600
ttgtttgatg tgcacagcat cctgcgggtg gtgctgggtg caaatggcac ctacagctgc 660
ctggtgcgca accccgtgct gcagcaggat gcgcacagct ctgtcaccat cacaccccag 720
agaagcccca caggagccgt ggaggtccag gtccctgagg acccggtggt ggccctagtg 780
ggcaccgatg ccaccctgcg ctgctccttc tcccccgagc ctggcttcag cctggcacag 840
ctcaacctca tctggcagct gacagacacc aaacagctgg tgcacagttt caccgaaggc 900
cgggaccagg gcagcgccta tgccaaccgc acggccctct tcccggacct gctggcacaa 960
ggcaatgcat ccctgaggct gcagcgcgtg cgtgtggcgg acgagggcag cttcacctgc 1020

ttcgtgagca tccgggattt cggcagcgct gccgtcagcc tgcaggtggc cgctccctac 1080
tcgaagccca gcatgaccct ggagcccaac aaggacctgc ggccagggga cacggtgacc 1140
atcacgtgct ccagctaccg gggctaccct gaggctgagg tgttctggca ggatgggcag 1200
ggtgtgcccc tgactggcaa cgtgaccacg tcgcagatgg ccaacgagca gggcttgttt 1260
gatgtgcaca gcgtcctgcg ggtggtgctg ggtgcgaatg gcacctacag ctgcctggtg 1320
cgcaaccccg tgctgcagca ggatgcgcac ggctctgtca ccatcacagg gcagcctatg 1380
acattccccc cagaggccct gtgggtgacc gtggggctgt ctgtctgtct cattgcactg 1440
ctggtggccc tggctttcgt gtgctggaga aagatcaaac agagctgtga ggaggagaat 1500
gcaggagctg aggaccagga tggggaggga gaaggctcca agacagccct gcagcctctg 1560
aaacactctg acagcaaaga agatgatgga caagaaatag cctga 1605

<210> 3
<211> 26
<212> PRT
<213> Homo sapiens

<400> 3
Ser Pro Thr Gly Ala Val Glu Val Gln Val Pro Glu Asp Pro Val Val
1               5                   10                  15

Ala Leu Val Gly Thr Asp Ala Thr Leu Arg
20

<210> 4
<211> 18
<212> PRT
<213> Homo sapiens

<400> 4
Asn Pro Val Leu Gln Gln Asp Ala His Ser Ser Val Thr Ile Thr Pro

1               5                   10                  15

Gln Arg

<210> 5
<211> 20
<212> DNA
<213> Artificial Sequence

<220>
<223> Description of Artificial Sequence:sense primer

<400> 5
accccgtgct gcagcaggat                                                20

<210> 6
<211> 20
<212> DNA
<213> Artificial Sequence

<220>
<223> Description of Artificial Sequence:antisense primer

<400> 6
atcctgctgc agcacggggt                                                20

<210> 7
<211> 2181
<212> DNA
<213> Artificial Sequence

<220>

<223> Description of Artificial Sequence: a fusion
      protein of BRIGHT extracellular domain (29-465)
      and human IgG1Fc domain

<400> 7
gaattcgcag ccttccacca cggggagccc agctgtcagc cgcctcacag gaagatgctg 60
cgtcggcggg gcagccctgg catgggtgtg catgtgggtg cagccctggg agcactgtgg 120
ttctgcctca caggagccct ggaggtccag gtccctgaag acccagtggt ggcactggtg 180
ggcaccgatg ccaccctgtg ctgctccttc tcccctgagc ctggcttcag cctggcacag 240
ctcaacctca tctggcagct gacagatacc aaacagctgg tgcacagctt tgctgagggc 300
caggaccagg gcagcgccta tgccaaccgc acggccctct tcccggacct gctggcacag 360
ggcaacgcat ccctgaggct gcagcgcgtg cgtgtggcgg acgagggcag cttcacctgc 420
ttcgtgagca tccgggattt cggcagcgct gccgtcagcc tgcaggtggc cgctccctac 480
tcgaagccca gcatgaccct ggagcccaac aaggacctgc ggccagggga cacggtgacc 540
atcacgtgct ccagctacca gggctaccct gaggctgagg tgttctggca ggatgggcag 600
ggtgtgcccc tgactggcaa cgtgaccacg tcgcagatgg ccaacgagca gggcttgttt 660
gatgtgcaca gcatcctgcg ggtggtgctg ggtgcaaatg gcacctacag ctgcctggtg 720
cgcaaccccg tgctgcagca ggatgcgcac agctctgtca ccatcacacc ccagagaagc 780
cccacaggag ccgtggaggt ccaggtccct gaggacccgg tggtggccct agtgggcacc 840
gatgccaccc tgcgctgctc cttctccccc gagcctggct tcagcctggc acagctcaac 900
ctcatctggc agctgacaga caccaaacag ctggtgcaca gtttcaccga aggccgggac 960
cagggcagcg cctatgccaa ccgcacggcc ctcttcccgg acctgctggc acaaggcaat 1020
gcatccctga ggctgcagcg cgtgcgtgtg gcggacgagg gcagcttcac ctgcttcgtg 1080
agcatccggg atttcggcag cgctgccgtc agcctgcagg tggccgctcc ctactcgaag 1140
cccagcatga ccctggagcc caacaaggac ctgcggccag gggacacggt gaccatcacg 1200
tgctccagct accggggcta ccctgaggct gaggtgttct ggcaggatgg gcagggtgtg 1260
cccctgactg gcaacgtgac cacgtcgcag atggccaacg agcagggctt gtttgatgtg 1320
cacagcgtcc tgcgggtggt gctgggtgcg aatggcacct acagctgcct ggtgcgcaac 1380

cccgtgctgc agcaggatgc gcacggctct gtcaccatca cagggcagcc tatgacattc 1440
cccccagagt ctagagcaga ctacaaggac gacgatgaca agactagtga caaaactcac 1500
acatgcccac cgtgcccagc acctgaactc ctggggggac cgtcagtctt cctcttcccc 1560
ccaaaaccca aggacaccct catgatctcc cggacccctg aggtcacatg cgtggtggtg 1620
gacgtgagcc acgaagaccc tgaggtcaag ttcaactggt acgtggacgg cgtggaggtg 1680
cataatgcca agacaaagcc gcgggaggag cagtacaaca gcacgtaccg tgtggtcagc 1740
gtcctcaccg tcctgcacca ggactggctg aatggcaagg agtacaagtg caaggtctcc 1800
aacaaagccc tcccagcccc catcgagaaa accatctcca aagccaaagg gcagccccga 1860
gaaccacagg tgtacaccct gcccccatcc cgggaggaga tgaccaagaa ccaggtcagc 1920
ctgacctgcc tggtcaaagg cttctatccc agcgacatcg ccgcggagtg ggagagcaat 1980
gggcagccgg agaacaacta caagaccacg cctcccgtgc tggactccga cggctccttc 2040
ttcctctata gcaagctcac cgtggacaag agcaggtggc agcaggggaa cgtcttctca 2100
tgctccgtga tgcatgaggc tctgcacaac cactacacgc agaagagcct ctccctgtct 2160
ccgggtaaat gatgagggcc c 2181

<210> 8
<211> 1463
<212> DNA
<213> Homo sapiens

<400> 8
gaattcgcag ccttccacca cggggagccc agctgtcagc cgcctcacag gaagatgctg 60
cgtcggcggg gcagccctgg catgggtgtg catgtgggtg cagccctggg agcactgtgg 120
ttctgcctca caggagccct ggaggtccag gtccctgaag acccagtggt ggcactggtg 180
ggcaccgatg ccaccctgtg ctgctccttc tcccctgagc ctggcttcag cctggcacag 240
ctcaacctca tctggcagct gacagatacc aaacagctgg tgcacagctt tgctgagggc 300
caggaccagg gcagcgccta tgccaaccgc acggccctct tcccggacct gctggcacag 360
ggcaacgcat ccctgaggct gcagcgcgtg cgtgtggcgg acgagggcag cttcacctgc 420
ttcgtgagca tccgggattt cggcagcgct gccgtcagcc tgcaggtggc cgctccctac 480
tcgaagccca gcatgaccct ggagcccaac aaggacctgc ggccagggga cacggtgacc 540

atcacgtgct ccagctacca gggctaccct gaggctgagg tgttctggca ggatgggcag 600
ggtgtgcccc tgactggcaa cgtgaccacg tcgcagatgg ccaacgagca gggcttgttt 660
gatgtgcaca gcatcctgcg ggtggtgctg ggtgcaaatg gcacctacag ctgcctggtg 720
cgcaaccccg tgctgcagca ggatgcgcac agctctgtca ccatcacacc ccagagaagc 780
cccacaggag ccgtggaggt ccaggtccct gaggacccgg tggtggccct agtgggcacc 840
gatgccaccc tgcgctgctc cttctccccc gagcctggct tcagcctggc acagctcaac 900
ctcatctggc agctgacaga caccaaacag ctggtgcaca gtttcaccga aggccgggac 960
cagggcagcg cctatgccaa ccgcacggcc ctcttcccgg acctgctggc acaaggcaat 1020
gcatccctga ggctgcagcg cgtgcgtgtg gcggacgagg gcagcttcac ctgcttcgtg 1080
agcatccggg atttcggcag cgctgccgtc agcctgcagg tggccgctcc ctactcgaag 1140
cccagcatga ccctggagcc caacaaggac ctgcggccag gggacacggt gaccatcacg 1200
tgctccagct accggggcta ccctgaggct gaggtgttct ggcaggatgg gcagggtgtg 1260
cccctgactg gcaacgtgac cacgtcgcag atggccaacg agcagggctt gtttgatgtg 1320
cacagcgtcc tgcgggtggt gctgggtgcg aatggcacct acagctgcct ggtgcgcaac 1380
cccgtgctgc agcaggatgc gcacggctct gtcaccatca cagggcagcc tatgacattc 1440
cccccagagt gatgagcggc cgc 1463

## Description

### TECHNICAL FIELD

The present invention relates to the membrane molecule, which is expressed in human dendritic cell (Dendritic Cell; hereinafter also referred to as "DC") and preferentially expressed in a mature DC, DNA encoding the membrane molecule, an antibody against the membrane molecule, and a method for separating mature DC and a method for detecting mature DC using the antibody.

### BACKGROUND OF THE INVENTION

Factors assumed to be involved in the onset of autoimmune diseases are (i) genetic background, (ii) exposure of autoantigens that are freed because of tissue inflammatory reaction and that are not naturally exposed to the immune system, and changes in a local-environment derived from inflammatory reaction due to viruses, bacteria, and the like, that is, changes in a local-environment resulting from, for example, activation of so called cross-reactive T cells that is due to homology of peptides to be recognized by activated T cells, and (iii) the presence of autoreactive T cells that are induced by a failure in tolerance due to abnormal expression of costimulatory molecules on cell membranes, and the like.

When antigen presenting cells are not yet stimulated in normal tissues, expression of co-stimulatory molecules is controlled even if autoantigens are presented. At this time, T cells are in the state of anergy, and are not activated, so that self-tolerance is maintained. However, it has been suggested that autoimmune diseases may be caused as a result of autoreactive T cells activated by excessive or continuous abnormal expression of co-stimulatory molecules under abnormal immune conditions. In particular, signals generated between CD 28/CD 152 and CD 80/CD 86 play an important and unique role in controlling T cell activation. Immunotherapy which involves regulation of these signals has been attempted in experimental mouse models and clinical trials of the immunotherapy have been started in humans.

Dendritic cells (DC) are known to differentiate and mature from CD34 positive cells that are the precursor cells of DC and exist in the bone marrow in vivo, and to play an important role as antigen presenting cells (APC) in induction, maintenance, extension and regulation of immune response. In the early 1990's, differentiation and induction of DC from the precursor cells became possible with cytokines. This enabled treatment of a large amount of DC, so that the importance of the role that DC play in immune response became clear at molecule, cell and in vivo levels, and DC began to attract attention as a target of immunoregulation.

It has been revealed from the results of past biological research that DC (monocyte-derived DC; also referred to as "mo-DC") can be induced by culturing human monocytes (also referred to as "Mo") in the presence of GM-CSF and IL-4 [J. Leucocyte Biol., vol. 59, pp208-218, (1996)]. It became clear that this in vitro differentiation and induction system actually has some functions of DC, though it is partially artificial. Important functions of DC are ingestion of antigens into cells (phagocytosis) and transmission of information of the antigens to T cells to stimulate and activate the T cells. Further, DC can be classified by the differentiation stages into two: immature DC and mature DC. Antigens ingested within immature DCs are subjected to processing within the cells, and then the peptides derived from the antigens are presented on MHC class II molecules of the DC surfaces. CD4 positive antigen-specific helper T cell recognizes with its antigen receptor a complex of antigen-derived peptide and MHC class II molecule, and is stimulated by co-stimulatory molecules at the same time, so that the CD4 positive antigen-specific T cell is sensitized and activated. Furthermore, CD8 positive cytotoxic T cells (CTL) are also stimulated and activated by DC via MHC class I molecule. Phagocytosis is strong in immature mo-DC and becomes weaker in mature mo-DC. The antigen presenting ability of immature mo-DCs to T cells is weak and that of mature mo-DC is strong in concert with the degree of expression of CD40, CD80, CD86, MHC class I molecule, and MHC class II molecule that are involved in antigen presentation.

It is easy to imagine that membrane molecules that are presented on the cell surface change with a functional change, from antigen-ingesting ability to antigen-presenting ability. However, when we consider what phenomena occur in this maturation process, the fact exists that molecules newly expressed on the surfaces of cell membranes are not always due to expression of mRNA. For example, it is known that HLA-DR being expressed at its immature stage translocates to the surfaces of cell membranes from the inside of the cells with almost no change in the expression level of mRNA and of protein as HLA-DR matures. In addition, there exist a case wherein almost no correlation is found when the actual expression level of mRNA and that of protein are compared, and a case wherein mRNA is expressed, but is not translated into protein [Biochem. Biophys. Res. Commun., vol. 231, pp 1-6, 1997]. To date, analysis has been energetically performed, because, for example, amplification from genes is possible, mass analysis is possible, handling is easy, and various techniques can be devised. However, it is also a fact that tracing changes in protein actually being expressed is more preferred, because such changes reflect better or more realistically the intracellular phenomenon.

As for DC, the presence of some subsets in DC has also been shown, in addition to the above findings. Such subsets are known to differ in functions depending on whether they are mature or immature subsets. However, the causes for such functional differences among subsets remain unclear. It is expected that at least membrane molecules thought to be involved in intracellular signaling, in particular, membrane molecules expressed with maturation of DC, will be elucidated.

As described above, DC, the strong APC existing in vivo, is known to highly express CD80/CD86 belonging to B7 family, as the DC matures with activation by stimulation. Regarding a relation with autoimmune diseases, for example, DCs existing in the synovial fluid of chronic articular rheumatism, the tissue of psoriasis lesions, and the dermal tissue of allergic contact dermatitis are known to abnormally express CD80/CD86.

CD28 has a property to strongly enhance activation of naive T cells. The presence of the CD28 signal enhances the production of IL-2 and expression of IL-2 receptor, thereby accelerating proliferation reaction. As a result, various effector functions of T cells are enhanced. The CD28 signal enhances not only IL-2, but also production of various cytokines such as IL-4, IL-5, IL-13, IFN-γ, TNF-α, and GM-CSF. The CD28 signal is also involved in expression of T cell activation antigen, such as CD40 ligands, and chemokines such as IL-8 and RANTES. There are many reports suggesting that binding of CD80 or CD86 to CD28 is involved in inducing CD4 positive T cells to differentiate into Th1 or Th2. However, the differentiation direction of naive CD4 positive T cells is not one-sidedly determined, such as CD80 involved in inducing differentiation into Th1 or CD86 involved in inducing differentiation into Th2. It is thought that the determination of differentiation direction is affected by various factors specified by antigens or the APC itself in addition to CD28 and CD152 signals. However, the fact that CD28 signals play an important role which is not compensated by the functions of other molecules, particularly in activation of differentiation of naive T cells into Th2, has been supported by the following results, for example: (i) the failure of selective production of IgG1 and IgG2b, the Th2 cytokine-dependent immunoglobulins, in CD28 knockout mice [Science, vol. 261, pp 609-612, 1993] and (ii) selective inhibition of differentiation into Th2 by antigen stimulation by DO11-10TCR transgenic mouse CD4 positive T cells and APC derived from CD80/CD86 double knockout mice [J. Immunol., vol. 161, pp 2762-2771, 1998]. Another important role of the CD28 signal is to enhance expression of a survival factor such as bcl-xL, so as to suppress apoptosis of T cells after stimulation with antigens.

Expression of CD80/CD86 is under control in tissue cells even though CD28 is expressed constantly on T cells. Specifically, the system works in such a way that even if T cells that react with autoantigens are present, excessive immune reaction does not occur. The failure of this tolerance induction system holds the hidden potential of causing various diseases such as autoimmune diseases and allergic diseases. Forced expression of CD80 or CD86 alone by islet β cells causes infiltration of inflammatory lymphocytes to the pancreas to be observed, but is not enough to cause the onset of insulin-dependent diabetes (IDDM) that is autoimmune diabetes. Only when major histocompatibility antigen complex (MHC) class II molecules, TNF-α, or viral proteins acting as autoantigens are co-expressed with CD80, the onset of IDDM associated with the failure of β cells can be induced. This suggests a possibility that the onset of autoimmune diseases is caused only when its environment is one that promotes reinforcement of T cell receptor (TCR) signals in addition to CD28-CD80/CD86 signals.

In some human organ-specific autoimmune diseases, expression of CD80/CD86 on professional APCs such as DCs and macrophages has been reported. As described above, CD80/CD86 are under control in APC in normal tissues. However, CD80/CD86 have been found in many cases to be abnormally expressed in the foci of diseases, suggesting a relationship with autoimmune diseases.

Currently, immunosuppressants that have many side effects and are non-specific are mainly used to treat autoimmune diseases. Inhibition of costimulation has effect of antigen specific suppression and prolonged suppression after administration. Reduction of the side effects by reducing the dose duration or maintenance of protective immune reaction against infectious diseases is expected. It is also expected that more effective therapies will be performed as the interaction and functions of cells of the immune system become clear. ICOS (inducible T cell co-stimulator) and PD-1 have been found as molecules of CD28 family that is expressed on activated T cells. It has been revealed that B7-H2 belonging to B7 family and B7-H1 are the ligands thereof, respectively. Elucidation of differences in costimulatory effects on T cells among APCs mediated by a plurality of B7 and CD28 families is now awaited.

We have focused on the maturation of DC, which is important in expression of the functions of DC. Specifically, we have conducted exploratory research centered on identification of membrane molecules that show significant increases in their expression levels with maturation of DC, based on comprehensive identification by proteomics of membrane molecules whose expression is regulated by maturation of DC. Proteomics means large-scale research conducted for protein, and is the term used for protein that corresponds to the term "genomics" used for genes. That is, the expression level of protein, and properties of post-translation modification, interaction and the like are studied. For example, overall biological information that is obtained by proteomics includes what occurs between normal cells and carcinoma cells at the expressed protein level, intracellular networks, processes, and the like.

Identification of membrane molecules specifically existing in DC enables preparation of an antibody against the membrane molecule, and the use of the antibody in the medical field.

The object of the present invention is to control autoimmune diseases and the like by regulating the functions of DC using as a target the molecule expressed on a mature DC that is the strong APC.

This molecule is inferred to be involved in activation of T cells, because it is expressed on APC, such as mature DC. Hence, other objects of the present invention are to provide a method for separating APC, such as mature DC, using an antibody that specifically recognizes the molecule, and a method for detecting mature DC.

### SUMMARY OF THE INVENTION

As a result of exploratory research on membrane molecules whose expression is regulated by maturation of DCs, we have identified a novel membrane molecule that is preferentially expressed on mature DCs to immature DCs at the protein level. This membrane molecule was shown to have homology with 4 types of molecules (B7-1, B7-2, B7-H1, B7-H2) that belong to the B7 family. The homology for the extracellular domain was around 25% (amino acid match) among the 4 types of molecules of B7 family. The molecule having the highest homology with this membrane molecule was B7-H1 and that is 31% homology for the extracellular domain. Further, this membrane molecule consisted of 4 immunoglobulin (Ig) domains in total that are two repetitive structures of two Ig domains that are characteristic in the B7 family. In this membrane molecule, the position of Cys residues involved in the S-S bond that is essential for maintaining the three-dimensional structure was conserved. Therefore, this membrane molecule was predicted to be a novel costimulatory molecule.

Recent researches have revealed that, 1) DCs are heterogeneous cell populations, and several DC subsets are present [Stem Cells, vol. 15, pp 409-419, 1997], 2) DC subsets have different functions (apoptosis induction, differential ability to differentiate T cells into Th1 and Th2, etc.) in activation of T cells [Science, vol. 283, pp 1183-1186, 1999], and 3) the regulation of immune response by targeting DCs (DC based immunotherapy for cancer, DC-specific drug, antibody, cytokine, etc.).

Human DC subsets are generally classified into myeloid DC and lymphoid DC. The myeloid DC has been shown to have two differentiation pathways [J. Exp. Med., vol. 184, 695-706, 1996]. It is known that CD34 positive hematopoietic stem cells cultured with GM-CSF and TNF-alpha differentiate into CD14 positive CD1a negative and CD14 negative CD1a positive precursor populations, and the former population differentiates into dermal DC, and the latter population differentiates into epidermal Langerhans cells. Mo-DCs can be related to the former population, and are thought to belong to the myeloid DC. On the other hand, the human cell population differentiated from plasma cell-like CD4 positive cells (CD11c negative, CD14 negative) in the presence of IL-3 is known to be lymphoid DC, and it becomes functionally mature DCs by the further stimulation with IL-3 and CD40 ligands [J. Exp. Med., vol. 185, pp 1101-1111, 1997]. Furthermore, it has been reported that mo-DCs when they are stimulation with CD40 ligands or endotoxin differentiate into, so-called DC1, having functions to differentiate naive T cells into Th1 cells [Science, vol. 283, pp 1183-1186, 1999]. As described above, it is understood that mo-DC that we have used can differentiate at least into DC1. Moreover, human peripheral blood DC (lineage markers (CD3, CD19, CD56 and CD14) negative and HLA-DR positive) consists of two subsets that are CD11c positive CD123 weakly positive and CD11c negative CD123 strong positive populations. After maturation of these DCs, the former population can be DC1, and the latter subset can be, so-called DC2, having functions to differentiate naive T cells into Th2 cells [Blood, vol. 95, pp 2484-2490, 2000].

Based on the findings that the expression of this membrane molecule significantly increases after maturation of mo-DC, it is thought to be involved in the activation of T cells and is also expected to be a target for manipulating immune response.

On the other hand, a pilot study of cancer vaccine has been performed using autologous antigen-pulsed peripheral blood DC [Nature Med., vol. 2, pp 52-58, 1996]. Based on the specificity of the expression of this membrane molecule, it can be a maker of the maturation of DC used for the cancer vaccines. Further, in such a case, the membrane molecule is also useful as a marker for the separation of mature DC from immature DC, and for the detection of mature DC, and the like.

Based on the above findings, the present invention is summarized as follows.
(1) An isolated human dendritic cell membrane molecule which has an amino acid sequence represented by SEQ ID NO: 1, or a variant thereof which has an amino acid sequence derived from the amino acid sequence by deletion, substitution, insertion and/or addition of one or more amino acid residues and is capable of regulating immune response.
(2) The variant described in (1) above containing an amino acid sequence from position 29 to 465 of SEQ ID NO: 1 corresponding to an extracellular domain.
(3) The variant of (2) above, which is a fusion protein of the protein having the amino acid sequence of position 29 to 465 of SEQ ID NO: 1 and another protein.
(4) The variant of (3) above, wherein the other protein is derived from a human.
(5) The variant of (3) above, which is a fusion protein of the protein having an amino acid sequence of position 29 to 465 of SEQ ID NO: 1 and a human IgGlFc domain.
(6) The human dendritic cell membrane molecule or the variant thereof of any one of (1) to (5) above, which has an effect to suppress proliferation and activation of T cells.
(7) A DNA which encodes the human dendritic cell membrane molecule or a variant thereof of any one of (1) to (6) above, or a complementary DNA thereof.
(8) The DNA or the complementary DNA thereof of (7) above, which contains a nucleotide sequence encoding an amino acid sequence of SEQ ID: 1 or a partial sequence thereof.
(9) The DNA or the complementary DNA thereof of (7) above, which contains a nucleotide sequence encoding an amino acid sequence of position 29 to 465 of SEQ ID NO: 1.
(10) The DNA or the complementary DNA thereof of (7) above, which has a nucleotide sequence selected from the group consisting of SEQ ID NO: 2, SEQ ID NO: 7 and SEQ ID NO: 8.
(11) An antibody or a fragment thereof, which specifically and immunologically binds to the human dendritic cell membrane molecule or a variant thereof of any one of (1) to (6) above or fragments thereof.
(12) The antibody or the fragment thereof of (11) above, which is characterized by recognizing an extracellular region of the above human dendritic cell membrane molecule.
(13) The antibody or the fragment thereof of (11) or (12) above, which is a polyclonal antibody, peptide antibody or monoclonal antibody.
(14) The antibody or the fragment thereof of any one of (11) to (13) above, wherein the above fragment is F(ab')₂.
(15) The antibody or the fragment thereof of any one of (11) to (14), which has an effect of enhancing proliferation and activation of T cells.
(16) The antibody or the fragment thereof of any one of (11) to (14) above, which has a characteristic of enhancing or suppressing proliferation of T cells depending on the cell ratio of T cells and dendritic cells.
(17) The antibody or the fragment thereof of any one of (11) to (14) above, which has the activity to suppress IgM production by B cells.
(18) A method for separating human-derived or other animals-derived mature dendritic cells using the antibody or the fragment thereof of any one of (11) to (17) above.
(19) A method for detecting mature dendritic cells using the antibody or the fragment thereof of any one of (11) to (17) above.

The term "specifically and immunologically binds" used in this specification concerning the antibody of the present invention means that the antibody of the present invention immunologically cross-reacts with an epitope that only the membrane molecule has, but does not cross-react with a protein having no such epitope. Such an epitope can be determined, for example, by comparing the amino acid sequence of the membrane molecule of the present invention with an amino acid sequence of another protein by aligning the sequences, so as to select a substantially different sequence portion (at least 5 consecutive amino acids, preferably at least 8 consecutive amino acids, and more preferably at least 15 consecutive amino acids).

The term "capable of regulating immune response" used in this specification can mean to be capable of activating T cells at a level identical to, or substantially equivalent to, or exceeding the level of the natural membrane molecule of the present invention, or can mean to be capable of suppressing or inhibiting activation of T cells.

### BRIEF DESCRIPTION OF DRAWINGS

Figure 1 shows a hydropathy plot performed for the primary structure of deduced amino acid sequence of the membrane molecule, according to the method of Kyte and Doolittle (J. Exp. Med., vol. 157, pp105-132, 1982).
Figure 2 shows the results of Western analysis performed using the peptide antibody of Example 7 for soluble recombinant BRIGHT and monocyte-derived DC (mo-DC).
Figure 3 shows the results of SDS-PAGE performed for BRIGHT-Ig under reducing conditions and non-reducing conditions.
Figure 4 shows the results of SDS-PAGE performed for BRIGHT-SF under reducing conditions and non-reducing conditions.
Figure 5 shows the results of in vitro activation of peripheral blood mononuclear cells and of expression analysis on the cell surface of BRIGHT as measured by flow cytometry.
Figure 6 shows the results of analysis on expression distributions of BRIGHT in human mo-DCs.
Figure 7 shows the results of analysis on BRIGHT counter receptors expressed on activated T cells.
Figure 8 shows the suppressive effect of BRIGHT-Ig on T-cell proliferation.
Figure 9 shows the suppressive effect of BRIGHT-Ig on allogeneic mixed leukocyte reaction.
Figure 10 shows the suppression of the expression of cytokine mRNA in allogeneic mixed leukocyte reaction by BRIGHT-Ig.
Figure 11 shows the suppression of cytokine secretion in allogeneic mixed leukocyte reaction by BRIGHT-Ig.
Figure 12 shows an induction of cell proliferation of human CD14 negative PBMC by anti-BRIGHT F(ab')₂.
Figure 13 shows the results of flow cytometry analysis on peripheral blood mononuclear cells of healthy individuals after the addition of anti-BRIGHT F(ab')₂.
Figure 14 shows an effect of anti-BRIGHT F(ab')₂ on T cell proliferation induced by super antigens.
Figure 15 shows the suppressive effect of anti-BRIGHT F(ab')₂ on IgM production from B cells induced with DC.

### DETAILED DESCRIPTION OF THE INVENTION

The present invention will now be described in further detail.

### Novel membrane molecule and DNA encoding the same

As described above, the present invention is based on the finding that the membrane molecule is expressed specifically on mature DC along with the maturation.

The human dendritic cell membrane molecule of the present invention was identified from the plasma membrane of mo-DC with or without the stimulation with lipopolysaccharide. The soluble proteins that had been prepared from plasma membrabe were fractionated by concanavalin A sepharose chromatography, wheat germ agglutinin sepharose chromatography, or SDS-polyacrylamide gel electrophoresis etc., and then applyied to microanalysis by LC/MS (in particular, LCQ manufactured by Thermoquest) (see Examples 1 to 5 described later). Furthermore, the gene fragments of the membrane molecule of the present invention were amplified and obtained by polymerase chain reaction (PCR) using a mature DC-derived cDNA library as a template with primers synthesized based on multiple partial amino acid sequences identified by LC/MS method. Then the clones containing the gene of the membrane molecule of the present invention were selected by colony hybridization using the gene fragments obtained above as probes, and then the nucleotide sequence (SEQ ID NO: 2) and the amino acid sequence (SEQ ID NO: 1) were determined (see Example 6 described later).

The novel membrane molecule of the present invention is predicted to consist of 525 or 534 amino acid residues as analyzed by hydropathy plot analysis (J. Exp. Med., vol. 157, pp105-132, 1982). Specifically, it is predicted to have a signal sequence and an extracellular region comprising 456 or 465 residues, 24 residues of a transmembrane region, and 45 residues of an intracellular region. Further, the extracellular region has 4 domains with the structures belonging to Ig super families containing 8 asparagine-linked sugar chain-binding sites, and 8 Cys residues required for forming Ig domains, as assessed by homology search and motif search. The extracellular Ig domain has a structure comprising V set, C set, V set and C set from the N-terminus, so that it was shown to be a novel molecule having a structure with repeated units of V set-C set.

The membrane molecule of the present invention can be synthesized in large quantities using gene cloning and DNA recombination technology. Examples of general techniques that can be used for such synthesis include standard techniques described in, for example, Sambrook et al., Molecular Cloning: A Laboratory Manual, 2nd Ed., Cold Spring Harbor Laboratory Press, Cold Spring Harbor, N.Y. (1989), and Ausubel et al., Current Protocols in Molecular Biology, Greene Publishing Associates and Wiley Interscience, N.Y. (1989).

Using the above standard techniques, mRNA is extracted from human mature DC, so that a cDNA library is constructed. cDNA of the membrane molecule of the present invention can be obtained by screening the library using a specific probe that has been synthesized based on the sequence represented by SEQ ID NO: 3 or 4. Alternatively, sense and antisense primers are synthesized to amplify sequences containing the mature sequence of a target molecule based on the sequence of SEQ ID NO: 3 or 4, and PCR is performed using the cDNA library as a template, so that the target cDNA can be amplified. PCR is preferably performed using an automated thermal cycler. PCR reaction can be performed in the presence of heat-stable polymerase (Taq or the like), a template DNA and primers, for approximately 25 to 40 cycles, followed by heating for 5 to 15 minutes at 70 to 75°C. Each PCR cycle consists of DNA denaturation (for example, at 94°C for 15 to 30 seconds), annealing of primers (for example, at 55°C for 30 seconds to 1 minute), and elongation reaction (for example, at 72°C for 30 seconds to 10 minutes) in the presence of four types of substrates (dNTP). The size of the primer is normally at least 15 nucleotides.

cDNA encoding the membrane molecule of the present invention that is inserted in an expression vector (for example, plasmids, phages, cosmids and viruses) containing an appropriate transcription/translation regulation sequence can be used for transformation or transfection or transduction of appropriate host cells (eukaryotic or prokaryotic cells).

The transcription/translation regulation sequence may contain a promoter and enhancer selected according to a host/vector system used herein. Examples of the promoter include P_{L}, P_{R}, Ptrp, Plac, and the like for a bacterial host/vector system, PHO5, GAP, ADH, AOX1 promoters and the like for a yeast host/vector system, and SV40 early promoter, retrovirus promoter, heat shock promoter, and the like for animal cell system.

Examples of the host cells include prokaryotic cells of, for example, the genus *Escherichia*, the genus *Bacillus* and the genus *Pseudomonas*, yeast of, for example, the genus *Saccharomyces* and the genus *Pichia*, and animal cells, for example, human fetal nephrocytes, human leukemia cells, African green monkey nephrocytes and Chinese hamster ovarian cells (CHO). In addition, insect cells and plant cells can also be used.

Expression vectors that can be used according to the host type are various vectors that are commercially available or have been deposited, or described in the relevant literature and other publications. Examples of the expression vector for bacteria include pQE (QIAGEN), pBluescript II SK+ (STRATAGENE) and pET (Novagen). Examples of a method for transforming or transfecting host cells using vectors include a method using calcium ion, an electroporation method, a protoplast method, and a microinjection method.

The transformed or transfected host cells are cultured in appropriate culture media to cause target genes to be expressed, and then the thus produced membrane molecules of the present invention are collected from the media or the host cells. To collect the membrane molecule from cells, after culturing, cells are separated by centrifugation or the like, suspended in an aqueous buffer solution, and then disrupted by sonication, french press, dynomill or the like, thereby obtaining an acellular extraction solution. The membrane molecule is isolated and purified by any combination of general methods employed for protein purification, for example chromatography such as gel filtration, ion exchange chromatography, affinity chromatography and hydrophobic chromatography, HPLC, electrophoresis, desalting method and organic solvent precipitation method.

The human dendritic cell membrane molecule and the variant thereof of the present invention have an effect of suppressing proliferation and activation of T cells (see Examples 18 to 21 described later).

### Variant

In addition to the human dendritic cell membrane molecule having the amino acid sequence as represented by SEQ ID NO: 1, the present invention also provides the variant of the membrane molecule which comprises an amino acid sequence derived from the amino acid sequence of SEQ ID NO: 1 by deletion, substitution, insertion and/or addition of one or more amino acid residues, and is capable of regulating immune response. The variant of the present invention preferably has 80% or more, particularly 90% or more, more preferably 95% or more, and most preferably 98% or more homology with the amino acid sequence of SEQ ID NO: 1, and preferably with the amino acid sequence of the extracellular region thereof. The term "homology" used in this specification means sequence identity or similarity between two or more amino acid sequences or nucleotide sequences. Sequences can be compared by any conventional method including a diagonal pattern method, a frequency distribution method and the like.

Any variant can be encompassed in the scope of the present invention, as long as it has high homology (preferably, 80% or more) with a natural membrane molecule, and is capable of regulating activation of T cells. Such a variant can be produced by introducing a desired alteration (deletion, substitution, insertion and/or addition) into the natural membrane molecule by the site-directed mutagenesis method, the PCR method or the like (see the above Sambrook et al., Ausubel et al., and the like). Examples of such an alteration include a substitution between conservative amino acids, for example, between acidic amino acids (asparatic acid and glutamic acid), between basic amino acids (lysin and arginine), and between hydrophobic amino acids (leucine, isoleucine, valine and the like).

An example of the variant of the present invention is a protein comprising an amino acid sequence of position 29 to 465 of SEQ ID NO: 1 corresponding to the extracellular domain.

Another example of the variant of the present invention is a fusion protein of the protein having the amino acid sequence of position 29 to 465 of SEQ ID NO: 1 and another protein. Another protein is preferably derived from a human, and is, for example, a human IgG1 Fc domain.

When the variant of the present invention is obtained from libraries or the like derived from a human or other animals such as a mouse, a probe is prepared based on the nucleotide sequence represented by SEQ ID NO: 2, hybridization is performed under moderate or highly stringent conditions, washing is performed under highly stringent conditions so as to extract a gene encoding the target variant, and the gene is inserted into an appropriate vector to express the gene, so that the target variant can be obtained. An example of the highly stringent conditions consists of hybridization with 0.5M NaHPO₄, 7% SDS and 1mM EDTA at 65°C, followed by washing with 0.1 × SSC/0.1% SDS at 68°C (see Ausubel et al., as described above). The hybridization conditions can be determined by appropriately selecting temperature, ionic strength, primer length and the like. Normally, the higher the temperature and the lower the ion strength, the higher the stringency. Thus, a person skilled in the art can select appropriate hybridization conditions.

The present invention also encompasses a DNA which comprises a nucleotide sequence encoding the amino acid sequence of SEQ ID NO: 1 or the partial sequence thereof, or the complementary DNA thereof. A specific example of the DNA or the complementary DNA thereof comprises a nucleotide sequence encoding an amino acid sequence of position 29 to 465 of SEQ ID NO: 1. More specifically, such a DNA has a nucleotide sequence selected from the group consisting of SEQ ID NO: 2, SEQ ID NO: 7 and SEQ ID NO: 8.

The present invention further encompasses a DNA encoding the above variant, which has preferably 80% or more, particularly 90% or more, more preferably 95% or more, and most preferably 98% or more homology with the nucleotide sequence represented by SEQ ID NO: 2, or the complementary DNA thereof.

### Antibody

It is predicted that the membrane molecule is also expressed in mature DC, activated macrophages, and activated monocytes, as presumed from the properties of B7 family. Antibodies against the membrane molecule that specifically recognizes activated antigen presenting cells (APC) can be obtained using this finding.

Any antibodies with the above properties can be encompassed in the present invention. An antigen epitope for obtaining a target antibody can be selected from a region with high antigenicity, a region with superficiality, a region which may not form a secondary structure, and a region with no homology or low homology with other proteins (particularly, the other proteins of B7 family), in the regions in the amino acid sequence (SEQ ID NO: 1) of the membrane molecule. The region with high antigenicity can be predicted by the method of Parker et al. [Biochemistry, vol. 25, pp5425-5432, 1986]. The region with superficiality can be predicted by, for example, calculating and plotting a hydropathy index. The region that may not form a secondary structure can be predicted by, for example, the method of Chou and Fasman [Adv. Enzymol. Relat. Areas Mol. Biol., vol. 47, pp 45-148, 1978]. Further, the region with no or low homology with, in particular, the other proteins of B7 family can be predicted by comparing the homology of the amino acid sequence of the membrane molecule with that of the amino acid sequence of the other protein.

Based on a partial amino acid sequence of the membrane molecule predicted by the above techniques, a peptide comprising the amino acid sequence can be synthesized using a peptide synthesis method. For example, a target peptide is synthesized using a commercial peptide synthesizer that was developed by R.B. Merrifield [Science, vol. 232, pp341-347, 1986] and which is based on solid phase peptide synthesis, protecting groups are removed, and the peptide is then purified by one of or a combination of ion exchange chromatography, gel filtration chromatography, reverse phase chromatography and the like. The thus purified peptide bound to a carrier protein such as key-hole-lympet hemocyanin (KLH) or albumin can be used as an immunogen.

Moreover, a polyclonal or monoclonal antibody against the membrane molecule can be prepared by known techniques using the gene recombinant membrane molecule as an immunogen. In this case, the term "recombinant" used for the membrane molecule, monoclonal antibodies, polyclonal antibodies, or other proteins means that these proteins are produced by recombinant DNA within host cells. Both prokaryotes (for example, bacteria such as *Escherichia coli*) and eukaryotes (for example, yeast, CHO cells, insect cells and the like) can be used as host cells.

The term "antibody" in the present invention may be any one of a peptide antibody, a polyclonal antibody and a monoclonal antibody. "Antibodies" can be obtained by immunizing a mouse or other appropriate animal hosts with antigens or antigen-expressing cells through the subcutaneous, intra-abdominal or intramuscular route in order to induce lymphocytes that produce or may produce antibodies that are thought to bind specifically to the protein used for this immunization. Further, a desired human antibody can also be obtained by administering antigens or antigen-expressing cells to host animals such as transgenic animals having human antibody gene repertoires [see Proc. Natl. Acad. Sci. USA, vol. 97, pp 722-727, 2000, International Publication WO96/33735, WO97/07671, WO97/13852 and WO98/37757]. Lymphocytes may also be immunized in vitro. Polyclonal antibodies can be obtained by collecting and purifying fractions that bind to antigens from the serum obtained from host animals. Further, monoclonal antibodies can be prepared by fusing lymphocytes with myeloma cells to form hybridoma cells using an appropriate fusion reagent such as polyethylene glycol (Goding, Monoclonal Antibodies: Principals and Practice, pp 59-103, Academic press, 1986). For example, the monoclonal antibody of the present invention can be prepared either by the hybridoma method [Nature, vol. 256, p 495, 1975], or the recombinant DNA method (Cabilly et al., US Patent No. 4816567).

Antigen proteins can be prepared by allowing DNA encoding the whole or partial sequence of the protein of the membrane molecule to be expressed in *Escherichia coli*, yeast, insect cells, animal cells or the like. The recombinant membrane molecule is purified by one of or a combination of methods including affinity chromatography, ion exchange chromatography, gel filtration chromatography, reverse phase chromatography and the like. The purified sample is used as an immunogen.

Further, the antibody of the present invention may be an intact antibody, or an antibody fragment such as (Fab')₂ or Fab.

Other antibodies that are also encompassed in the antibody of the present invention are: chimeric antibodies wherein a constant region is substituted with a human constant region (for example, mouse-human chimeric antibody; Cabilly et al., US Patent No. 4816567 and Morrison et al., Proc. Natl. Acad. Sci. USA, vol. 89, p 6851, 1984); and humanized antibodies wherein all the variable regions excluding constant regions and hypervariable regions (or Complementary-determining region: CDR) are substituted with human sequences (Carter et al., Proc. Natl. Acad. Sci. USA, vol. 89, p 4285, 1992, and Carter et al., BioTechnology, vol. 10, p 163, 1992).

In addition, the present invention also encompasses antibodies against the thus obtained antibodies of the present invention, namely, anti-idiotype antibodies.

The thus obtained various antibodies against the membrane molecule can be used for various applications that can make use of the characteristics. Utilizing the fact that the membrane molecule is expressed specifically on mature DC, the antibody labeled with fluorescent substances (rhodamine, fluorescamine and the like) can be used to detect or separate target DC with known FACS, and to confirm in vitro differentiation of mo-DC. Further, the membrane molecule is a protein showing significant increases in expression levels with the maturation of DC. Thus, immature DC can be separated from mature DC with FACS using the antibodies. Further, detection of this membrane molecule is not limited to detection with FACS. For example, it is predicted that detection is possible by using the antibodies as primary antibodies in the Western blotting, and expression can also be confirmed at the protein level. Further, it is possible to bind the antibodies to solid phase (polystyrene beads, microtiter well surface, latex beads and the like), and then perform immunological reaction in a heterogeneous system or homogeneous system, so that homologous membrane molecules can be detected and quantitatively determined (fluorescence antibody method, ELISA, radioimmunoassay or the like is used). In this case, the immunological reaction may be competitive reaction or non-competitive reaction. Further, reaction by the Sandwich method using two or more antibodies (monoclone or polyclone) can be used. For the detection and quantitative determination as described above, any immunological techniques known in the art can be used.

In addition, the antibody can also be used for applications to assess the functions of the membrane molecule. Mature DC, the strong APC, is known to stimulate and activate CD4 positive T cells through MHC class II molecules, and stimulate and activate CD8 positive cytotoxic T cells through MHC class I molecules. To confirm whether or not these functions can be regulated, the antibody can also be used for in vitro assay to confirm whether or not the functions in allogeneic MLR (mixed leukocyte reaction) can be suppressed, whether or not the functions can be suppressed in the case of antigen-specific induction of CTL, whether or not molecules are involved in antigen presentation by DC, and the like.

The antibody of the present invention can be further used to regulate in vivo immune response. The membrane molecule of the present invention is, as described above, predicted to be a costimulatory molecule, and to be a membrane molecule involved directly or indirectly in T-cell activation. As shown in Examples 18 to 21 to be described later, the membrane molecule of the present invention has an effect of suppressing T-cell proliferation and T-cell activation. Accordingly, if the antibody of the present invention inhibits the binding of the membrane molecules to counter receptors on cells, the membrane molecule is predicted to enhance the activation of T cells. Actually, in Examples 22 and 23 to be described later, it was confirmed that the antibody enhances the proliferation and activation of T cells. On the other hand, in an experiment of autologous mixed leukocyte reaction in Example 24, it was confirmed that the antibody enhances or, conversely, suppresses T-cell proliferation, depending on the mixture ratio of T cells and DCs. This suggests that the antibody of the present invention is useful in a way whereby it enhances or suppresses the proliferation or activation of T cells. Further, as shown in Example 25, the antibody of the present invention also has activity to suppress IgM production by B cells. As described above, it is expected that immune response can be regulated by allowing the antibody capable of regulating the functions of the membrane molecule of the present invention to regulate the functions of DC and, further, the functions of T cells.

Moreover, it is also expected that the membrane molecule, the soluble molecule of the counter receptor of the membrane molecule (namely, the molecule corresponding to the extracellular region), and antibodies against these molecules have activities to regulate immune response.

The counter receptors of the membrane molecules can also be obtained using antibodies in various forms or the soluble molecule of the membrane molecule of the present invention. The counter receptor of the soluble membrane molecule can regulate signals through the membrane molecule on DC by directly acting on the membrane molecule, and can also block signals through counter receptors expressed on cells. Further, low molecular substances that can modulate interaction between the counter receptor of the membrane molecule and the membrane molecule, and low molecular substances that modulate an intracellular signal pathway involved in the counter receptor of the membrane molecule and the membrane molecule can also be useful in regulation of signals.

The antibodies in various forms or the soluble molecule of the membrane molecule, the soluble molecule of the counter receptor of the membrane molecule, and the above low molecule of the present invention can be applied to medical treatment in areas including organ transplantation and treatment of diseases such as cancer, autoimmune diseases, infectious diseases and allergy.

The route of administration and dosage form are not specifically limited. Examples of the route of administration include intravenous, intraarterial administration, intramuscular administration, oral administration and suppository administration. Further, the above antibodies and membrane molecules may be formulated in combination with a pharmaceutically acceptable excipient and diluent for oral or parenteral administration. Parenteral administration is preferred. The dose is administered once or several times per day. The dose is determined depending on conditions including severity of condition, age, sexuality, weight and the like of a patient, and is within the rate that causes no side effect.

### EXAMPLE

Examples of the present invention are described hereunder, but the present invention is not limited by these Examples.

### Example 1 Preparation of DC cell membrane

Problems involved in preparation of a cell membrane protein include its originally low expression level and difficulty in its handling. As a means to solve these problems, it is necessary to establish a method for obtaining cell membrane with higher purity. DC cell membrane of high purity was prepared by coating the cell membrane surface, disrupting uniformly the coated surface, and then obtaining the cell membrane with higher density by density gradient centrifugation [J. Biol. Chem., vol. 258, pp 10062-10072 (1983)].

Since a target membrane molecule has a low expression level and is difficult to handle, monocytes were separated from apheresis product (mononuclear leukocyte fraction) in order to collect a large amount of human DCs, and then mo-DC was prepared in large numbers. Some of the cells were stimulated with LPS (lipopolysaccharide), and an equivalent number of the cells was not stimulated with the same. Thus, immature DCs and mature DCs were obtained in the same number (5 x 10⁸ cells).

### Example 2 High sensitivity detection of protein and in gel digestion

Protein was detected by silver staining according to the technique of Mann et al. [Anal. Chem., vol. 68, pp 850-858, 1996]. The in gel digestion method [Anal. Chem., vol. 224, pp 451-455, 1995] using trypsin was performed as an enzymatic digestion method to obtain analytical samples to be used below.

### Example 3 Fractionation of membrane protein

After the cell membrane protein obtained from the cell membrane in Example 1 was solubilized using a detergent, the cell membrane protein was applied to concanavalin A sepharose, and then washed in a detergent-containing buffer. The fractions that passed through were named ConA FT. The adsorbed fractions were eluted with a buffer containing methyl-alpha-D-glucopyranoside and surfactant (ConA EL). ConA FT was applied again to wheat germ agglutinin sepharose, and then washed in a surfactant-containing buffer. The fractions that passed through were named WGA FT. The adsorbed fractions were eluted with a buffer containing N-acetylglucosamine and surfactant (WGA EL). ConA EL, WGA EL and WGA FT were used as samples for molecule identification. After these fractions were applied to SDS-PAGE, protein was detected by the techniques of Example 2, and then gel cut into a strip shape was digested in gel (in gel digestion), thereby preparing analytical samples.

### Example 4 Microanalysis by LC/MS

The samples obtained in Example 3 were analyzed using LC/MS (LCQ, Thermoquest). The samples were applied to PepMap reversed-phase column (0.075 mm in internal diameter × 150 mm in length) (LC packings) that had been equilibrated with 95% solution A (0.1% formic acid) and 5% solution B (0.08% formic acid, 80% acetonitrile), washed for 5 minutes at a flow rate of 180 nl/minute, eluted sequentially by linearly raising the proportion of solution B to 50% for 67.5 minutes, and then introduced into MS. Data were acquired for the samples introduced into MS with the following repetitive cycle, thereby obtaining the sequence information. 1. Full MS Scan: Observation of molecular weight of parent ion with a range of m/z = 0 to 2000. 2. Zoom MS Scan: Observation of the valence of parent ion identified with Full MS Scan. 3. MS/MS Scan: Observation of daughter ion generated when He gas is applied to the molecule measured with Zoom MS Scan. Identification was performed by an identification method (SEQUEST algorithm) [American Society for mass spectrometry, vol. 5, pp 976-989, 1994] that scores and ranks how many sequences match with theoretical b,y series at which intensities.

### Example 5 Identification of the membrane molecule

Among the molecules identified by the procedure in Example 4, the membrane molecules, i.e. the novel molecules, were identified only from mature DC. Fragments subjected to identification were divalent ion of m/z = 1310.8 and divalent ion of m/z = 995.8. The MS/MS patterns were attributed extremely well to a partial amino acid sequence (26 residues) SPTGAVEVQVPEDPVVALVGTDATLR (SEQ ID NO: 3) and a partial amino acid sequence (18 residues) NPVLQQDAHSSVTITPQR (SEQ ID NO: 4) of the membrane molecule. As a result of a homology search for these partial sequences on the non-redundant DNA database, it was shown that no other nucleotide sequence having these partial sequences was present.

### Example 6 Gene cloning of the membrane molecule

The two partial amino acid sequences identified in Example 5 were searched for on the human genome database, revealing that they were genes on AC022188. Further, the gene of this membrane molecule was estimated to have duplication on the genome, showing that the partial amino acid sequences identified in Example 5 were present on a duplicated portion. Then, primers were synthesized based on gene sequences corresponding to the partial amino acid sequences shown in Example 5, a mature DC cDNA library was used as a template, and RT-PCR was then performed. Primers used herein were respectively 20 mer: a sense primer 5'-ACCCCGTGCTGCAGCAGGAT-3' (SEQ ID NO: 5) and an antisense primer 5'-ATCCTGCTGCAGCACGGGGT-3' (SEQ ID NO: 6). As a result, a gene fragment of the membrane molecule of about 800 bp was obtained. Colony hybridization was performed using the gene fragment as a probe, and then clones containing the gene of the membrane molecule were selected, thereby determining the nucleotide sequence. The deduced open reading frame (ORF) structure is shown in SEQ ID NO: 2, and the deduced amino acid sequence is shown in SEQ ID NO: 1.

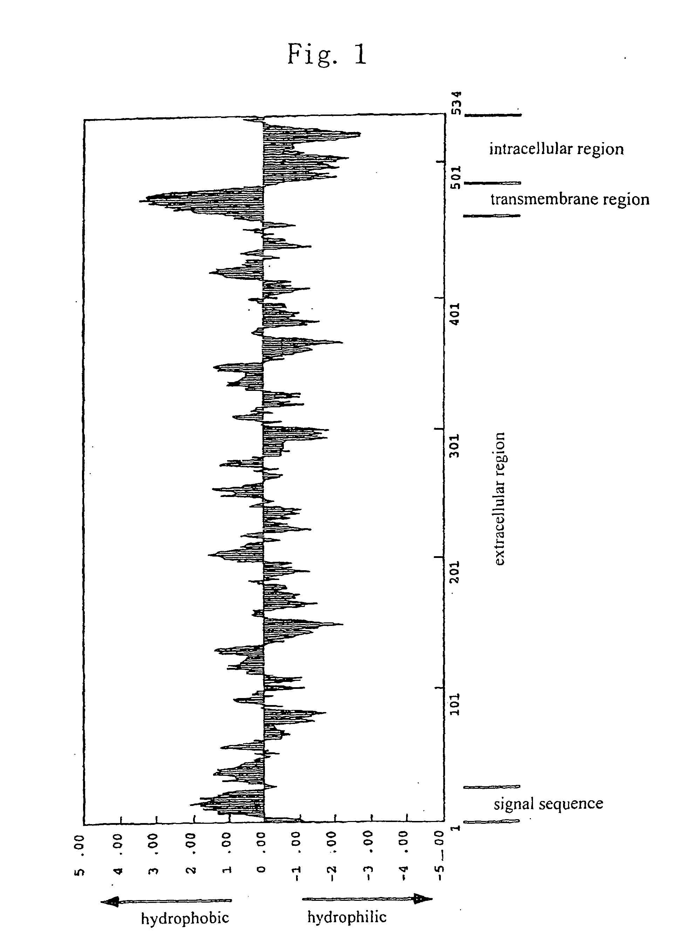

The primary structure of the deduced amino acid sequence of the membrane molecule was subjected to hydropathy plot analysis according to the method of Kyte and Doolittle (J. Exp. Med., vol. 157, pp 105-132, 1982) (Fig. 1). As a result, it was revealed that the membrane molecule was a type I transmembrane protein having a signal sequence at the N-terminus. The membrane molecule consisted of 534 amino acid residues, and was predicted to contain a signal sequence (position 1 to 28 of SEQ ID NO: 1) and an extracellular region (position 29 to 465 of SEQ ID NO: 1) consisting of 465 residues, a transmembrane region of 24 residues, and an intracellular region of 45 residues as a result of hydropathy plot analysis. Further, homology search and motif search revealed that the extracellular region has four domains with the structure belonging to immunoglobulin super families having 8 asparagine-linked sugar chain-addition sites, and cysteine residues necessary for the formation of 8 immunoglobulin (Ig) domains. In addition, the extracellular Ig domain was predicted to have a structure of V set, C set, V set, C set from the N terminal side. That is, it had a structure of repetition of a V set-C set unit, and had 95% homology at the amino acid sequence level. Further, a molecule having the highest homology in terms of the V set-C set unit was B7-H1 (B7-homologue 1), which had 31% of the amino acid match. The extracellular V set-C set unit is a common structure among B7 family. However, there has been no known molecule like the membrane molecule having the structure wherein the V set-C set unit is repeated, and has homology at the primary sequence level with B7 family.

### Example 7 Design of peptide antibody against membrane molecule and peptide synthesis and preparation of peptide antibody

To prepare a specific peptide antibody against the membrane molecule, a peptide antibody was designed from the deduced amino acid sequence of the membrane molecule. First, portions with high antigenicity were selected according to Parker's law (as described above). From these portions, single peptide was synthesized based on the method of Chou and Fasman (as described above) corresponding to portions with superficiality, portions that may not take secondary structure, portions that are not predicted to be added with sugar chain, and portions that do not contain cysteine residues. A selected region was a sequence consisting of the following amino acid positions: SPTGAVEVQVPEDPVVALVGTDATLR (position 242 to 267 of SEQ ID NO: 1). The single peptide was purified, and then allowed to conjugate at a concentration of 0.2 mg/ml with 1 mg/ml Keyhole limpet hemocyanin (KLH). Rabbits were repeatedly immunized 8 times with the KLH-conjugated peptide (100 µg) as an immunogen, so that peptide antibodies were prepared.

### Example 8 Naming of the membrane molecule and inferring of the deduced structure

The membrane molecule was named BRIGHT (B7 Related Ig Superfamily Homologue Transmembrane Molecule) , since it structurally belonged to B7 family of Ig super family. Based on the amino acid sequence prediction of a mouse counterpart and N-terminal amino acid sequence analysis in Example 10 to be described later, BRIGHT was expected to be a type I membrane protein consisting of 534 amino acid residues, and to have a signal sequence of 28 residues, an extracellular region of 437 residues, a transmembrane region of 24 residues, and an intracellular region of 45 residues.

### Example 9 Purification of peptide antibody and evaluation by Western analysis

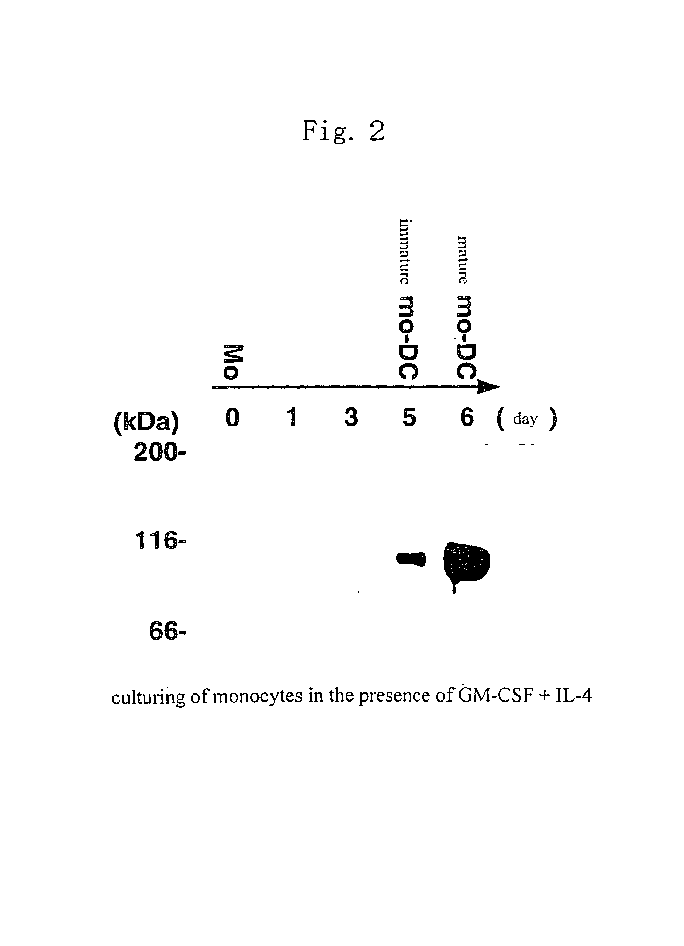

The titer of the antiserum of the rabbit immunized in Example 7 against the peptide of position 242 to 267 of SEQ ID NO: 1 increased 200000-fold or more. This antiserum was applied to Protein G sepharose (Amersham Pharmacia Biotech) so as to cause specific binding of IgG only, IgG bound sepharose was washed with PBS, and then the bound IgG was eluted with 0.1M glycine hydrochloric acid (pH 2.8). The eluate was quickly neutralized by adding 1M Tris hydrochloric acid (pH 7.5). The neutralized IgG fraction was concentrated, followed by substitution with PBS by gel filtration chromatography using Superdex 200 pg (10 mm in internal diameter × 300 mm in length) (Amersham Pharmacia Biotech), thereby purifying samples. Western analysis was performed for soluble recombinant BRIGHT (described in Example 11) and mo-DC (monocyte-derived DC), using the obtained peptide antibody sample. The sample was boiled (95°C for 5 minutes) under a reducing condition using 4/20 gradient gel (Daiichi Pure Chemicals). Then, the sample was electrophoresed at a constant current of 25 mA per piece of gel for 1.5 hours, and then transferred to PVDF membrane at a constant current of 150 mA per piece of gel for 1 hour. The transferred PVDF membrane was blocked with Block Ace (Snow Brand Milk Products) or the like, and then allowed to react with the peptide antibody as a primary antibody at a concentration of 2 µg/ml and HRP conjugated anti rabbit IgG (DAKO) as a secondary antibody. Thus, it was found that BRIGHT could be detected using a HRP coloring reagent, such as SuperSignal (Pierce). The monocyte-derived DC (mo-DC) was detected to be a broad band in the vicinity of 100 kDa that increased depending on the maturation of DCs. (See Fig. 2)

### Example 10 Production of soluble recombinant BRIGHT by CHO cell

The following two types (i) and (ii) of BRIGHT soluble recombinants were expressed in CHO cells: (i) a fusion protein of BRIGHT extracellular domain (position 29 to 465 of SEQ ID NO: 1) and human IgG1Fc domain (referred to as BRIGHT-Ig), and ii) BRIGHT extracellular domain (position 29 to 465 of SEQ ID NO: 1) (referred to as BRIGHT-SF). In the case of BRIGHT-Ig, a gene represented by SEQ ID NO: 7 was inserted between *Eco* RI site and *Apa* I site, and in the case of BRIGHT-SF, a gene represented by SEQ ID NO: 8 was inserted between *Eco* RI site and *Not* I site of an animal cell expression vector, pTracer CMV (Invitrogen). Thus, recombinant *Escherichia coli* was prepared and plasmids were prepared in large amount. The prepared plasmid genes were introduced into CHO cells in Opti-MEM culture media using a transfection reagent IT-LT1 (Mirus). BRIGHT expression cells were subjected to single cell sorting with FACS Vantage (Becton Dickinson) using GFP expression as an indicator, so that Zeocin (Invitrogen) (drug)-resistant clones were obtained. The supernatant of the culture product obtained after culturing in serum-free DF media for 3 days was subjected to Western analysis using peptide antibodies, so that strains with high expression levels were obtained.

### Example 11 Purification of BRIGHT-Ig and BRIGHT-SF

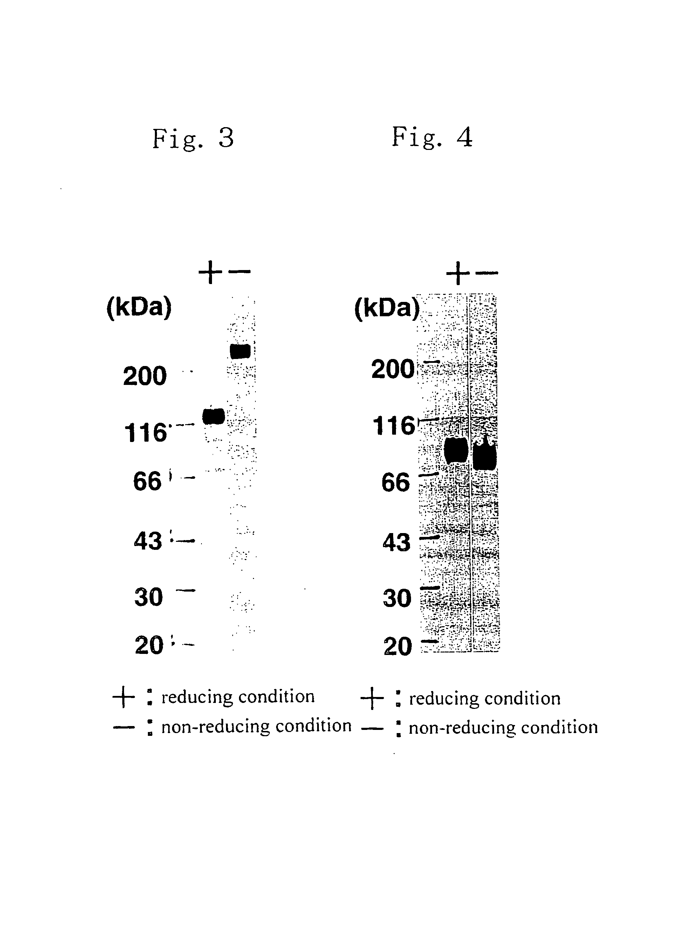

BRIGHT-Ig: Serum-free DF culture supernatant was concentrated to 10-fold using ultrafiltration membrane (YM10, Millipore), BRIGHT-Ig only was allowed to specifically bind to ProteinG sepharose (Amersham Pharmacia Biotech), BRIGHT-Ig-bound sepharose was washed with PBS, and then the bound BRIGHT-Ig was eluted with 0.1M glycine hydrochloric acid (pH 2.8). The eluate was quickly neutralized by adding 1M Tris hydrochloric acid (pH 7.5). The neutralized BRIGHT-Ig fraction was concentrated, followed by substitution with PBS by gel filtration chromatography using Superdex 200 pg (10 mm in internal diameter × 300 mm in length) (Amersham Pharmacia Biotech), thereby purifying samples. BRIGHT-Ig samples were detected by SDS PAGE as a 200 to 220 kDa band under a non-reducing condition, and as a broad 100 to 120 kDa band under a reducing condition (Fig. 3).

BRIGHT-SF: The serum-free DF culture supernatant was concentrated to 10-fold using ultrafiltration membrane (YM10, Millipore), and then BRIGHT-SF was allowed to bind to WGA sepharose (Amersham Pharmacia Biotech). After washing with PBS, bound BRIGHT-SF was eluted using PBS+0.1M N-acetyl glucosamine. The eluate was substituted with 20 mM Tris hydrochloric acid (pH 8.0) by dialysis, followed by fractionation by anion exchange chromatography. The fractions were applied to DEAE-5PW (4.6 mm in internal diameter × 150 mm in length) (TOSOH) equilibrated with 100% solution A (20 mM Tris hydrochloric acid (pH 8.0)) and 0% solution B (20 mM Tris hydrochloric acid (pH 8.0), 0.5M sodium chloride), at a flow rate of 1 ml/minute, washed for 5 minutes, and then sequentially eluted by linearly raising the proportion of solution B to 50% for 50 minutes. An equivalent volume of 2.5 M ammonium sulfate was added to the eluted BRIGHT-Ig fraction, and then fractionation was performed by hydrophobic chromatography. The fractions were applied to Phenyl-5PW (4.6 mm in internal diameter × 150 mm in length) (TOSOH) equilibrated with 100% solution A (20 mM Tris hydrochloric acid (pH 6.8), 1.2M ammonium sulfate) and 0% solution B (20 mM Tris hydrochloric acid (pH 6.8)), at a flow rate of 1 ml/minute, washed for 10 minutes, and then sequentially eluted by linearly raising the proportion of solution B to 100% for 50 minutes. The eluted BRIGHT-Ig fraction was concentrated, followed by substitution with PBS by gel filtration chromatography using Superdex 200 pg (10 mm in internal diameter × 300 mm in length) (Amersham Pharmacia Biotech), thereby purifying samples. BRIGHT-SF samples were detected by SDS-PAGE as a 65 to 80 kDa band under a non-reduction condition, and as a broad 70 to 90 kDa band under a reducing condition (See Fig. 4).

Both recombinants, BRIGHT-Ig and BRIGHT-SF, were confirmed to be soluble recombinants based on the results that specific staining was seen by Western analysis using anti-BRIGHT peptide antibodies, and that an amino acid sequence encoded by BRIGHT, wherein Leu residue at position 29 was the N-terminal amino acid, was obtained for both recombinants as revealed by N-terminal amino acid sequence analysis using an amino acid sequencer (Model 377, Perkin Elmer).

### Example 12 Preparation and purification of anti-BRIGHT polyclonal antibody

Rabbits were immunized repeatedly (8 times) with BRIGHT-SF (100 µg) as an immunogen, so that anti-BRIGHT polyclonal antibodies (hereinafter, referred to as anti-BRIGHT pAb) were prepared. The titer of the antisera of the immunized rabbits against BRIGHT-SF increased 200000-fold or more. These antisera were applied to Protein G sepharose (Amersham Pharmacia Biotech), so that only IgG was specifically bound. After washing with PBS, the bound IgG was eluted with 0.1 M glycine hydrochloric acid (pH 2.8). The eluate was quickly neutralized by adding 1 M Tris hydrochloric acid (pH 7.5). The neutralized IgG fraction was concentrated, followed by substitution with PBS by gel filtration chromatography using Superdex 200 pg (10 mm in internal diameter × 300 mm in length) (Amersham Pharmacia Biotech), thereby purifying samples.

At the same time, sera were obtained from normal rabbits as a control antibody, and IgG fraction was prepared by similar purification procedures, so that samples were purified (hereinafter, referred to as normal rabbit IgG).

### Example 13 Preparation of anti-BRIGHT pAb F(ab')₂ fragment and biotin-labeled anti-BRIGHT pAb F(ab')₂ fragment

F(ab')₂ fragment was obtained by digesting with pepsin anti-BRIGHT pAb and normal rabbit IgG obtained in Example 12. Specifically, 1 mg/ml anti-BRIGHT pAb or normal rabbit IgG was digested overnight with 0.1 mg/ml pepsin in 20 mM sodium acetate (pH 4.5) at 37°C. The anti-BRIGHT pAb F(ab')₂ fragment (hereinafter, referred to as anti-BRIGHT F(ab')₂) or normal rabbit IgG F(ab')₂ fragment (hereinafter, referred to as normal rabbit IgG F(ab')₂) obtained by the above procedure was concentrated. Then, substitution with PBS was performed by gel filtration chromatography using Superdex 200 pg (10 mm in internal diameter × 300 mm in length) (Amersham Pharmacia Biotech), while undigested and other digested fragments and pepsin were removed, thereby preparing anti-BRIGHT F(ab')₂ and normal rabbit IgG F(ab')₂ authentic samples.

The anti-BRIGHT F(ab')₂ and normal rabbit IgG F(ab')₂ can be labeled by reaction with a biotinylation reagent. After substitution with 10 mM HEPES-NaOH (pH 8.5), 2 µl of 10 mM Biotin-AC₅-Sulfo-OSu (DOJINDO) was added to anti-BRIGHT F(ab')₂ or normal rabbit IgG F(ab')₂ (2 mg/ml, 1 ml), and then the solution was allowed to react for 1 hour under ice cooling. Next, the solution was applied to gel filtration chromatography using Superdex 200 pg (10 mm in internal diameter × 300 mm in length) (Amersham Pharmacia Biotech) for substitution with PBS, thereby preparing biotin-labeled anti-BRIGHT F(ab')₂ (hereinafter, referred to as anti-BRIGHT F(ab')₂-biotin) and biotin-labeled normal rabbit IgG F(ab')₂ (hereinafter, referred to as normal rabbit IgG F(ab')₂-biotin) authentic samples.

### Example 14 Separation of peripheral blood mononuclear cells from healthy individual

Peripheral blood was collected from healthy individuals into a blood collection bag (TERUMO) containing a CPD solution to prevent clotting, and then centrifuged (600 G, at room temperature for 5 minutes) to separate a blood cell fraction from blood plasma. The blood cell fraction (excluding blood plasma) was diluted with PBS, layered on Ficoll-Paque (Amersham Pharmacia Biotech), and then applied to density gradient centrifugation (400 G, at room temperature for 30 minutes), so as to separate mononuclear cells. The erythrocytes contained together with the mononuclear cells were hemolyzed by treating with an ammonium chloride buffer (0.83% NH₄Cl-Tris HCl 20 mM, pH 6.8) at room temperature for 2 minutes, and then the mononuclear cells were washed with 5% FCS-containing PBS (hereinafter referred to as PBS-FCS). This cell population was used as peripheral blood mononuclear cells of healthy individuals.

### Example 15 In vitro activation of peripheral blood mononuclear cells and analysis of expression of BRIGHT on cell surface by flow cytometer

The peripheral blood mononuclear cells separated by the method of Example 14 were suspended to 2.5 × 10⁶ cells/ml in a 10% FCS containing RPMI 1640 medium (GIBCO BRL), and then inoculated in a 6-well culture plate (#3046, Falcon). PHA (SEIKAGAKU CORPORATION) at a final concentration of 5 µg/ml or LPS (SIGMA) at a final concentration of 1 g/ml or recombinant human GM-CSF (Kirin Brewery) at a final concentration of 50 ng/ml + recombinant human IL-3 (Kirin Brewery) at a final concentration of 50 ng/ml or PMA (SIGMA) at a final concentration of 5 ng/ml + Ionomycin (SIGMA) at a final concentration of 250 ng/ml was added to the medium, and then cultured for 2 days.

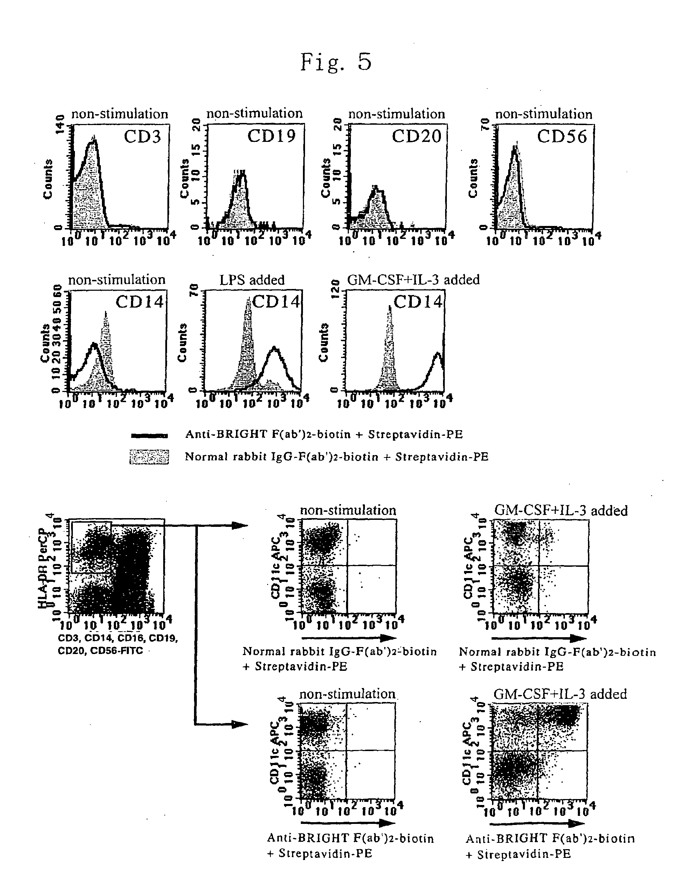

The peripheral blood mononuclear cells or the same activated in vitro were washed with PBS-FCS, and then suspended in 1 ml of PBS (hereinafter referred to as PBS-FCS-EDTA-NaN₃) containing 5% FCS, 10 mM EDTA, and 0.05% sodium azide. 100 µg of human IgG was added, and then incubated for 10 minutes on ice. Next, 20 µg of anti-BRIGHT F(ab')₂-biotin was added, and then incubated for 30 minutes on ice. 20 µg of normal rabbit IgG F(ab')₂-biotin was added to the cells of a control group, and then incubated for 30 minutes on ice. After the cells were washed with PBS-FCS-EDTA-NaN₃, PE (Phycoerythrin)-coupled streptavidin (BD Pharmingen) was added, and then the mixture was incubated for 30 minutes on ice. After the cells were washed with PBS-FCS-EDTA-NaN₃, FITC (fluorescein isothiocyanate) or APC (Alophicocyanine) or PerCP-labelled antibodies against antigens specific to human differentiated blood cells, namely, antibodies against CD3, CD11c, CD14, CD16, CD19, CD20, CD56, CD123, and HLA-DR (BD Pharmingen) were added, followed by incubation for 20 minutes on ice. Next, expression of antigens specific to BRIGHT and human cultured blood cells was analyzed using a flow cytometer (Becton Dickinson). Expression of BRIGHT was not observed on the cell membranes of peripheral blood mononuclear cell populations respectively expressing CD3, CD14, CD19, CD20, and CD56, but was observed on CD14 positive cells activated with LPS and recombinant human GM-CSF + recombinant human IL-3. Further, expression was not observed among a peripheral blood mononuclear cell population that is thought to differentiate into DC1, namely, expression was not observed in lineage marker (CD3, CD14, CD16, CD19, CD20, and CD56) negative, HLA-DR positive, CD11c positive cells, but increased expression was observed in the cell population when the cells were cultured in vitro with recombinant human GM-CSF + recombinant human IL-3 (see Fig. 5).

### Example 16 Analysis of expression distribution of BRIGHT in human monocyte-derived DC

Peripheral blood mononuclear cells collected from healthy individuals and prepared by density gradient centrifugation with Ficoll-Paque were suspended to 1 × 10⁸ cells/ml in PBS (PBS-plasma) containing 2% human plasma. Anti-human CD14 antibody-binding magnetic microbeads (Miltenyi Biotec) were added to the suspension, and then incubated for 30 minutes on ice. After the cells were washed with PBS-plasma, the cell suspension was applied to a separation column LS+ (Miltenyi Biotec) within a magnetic field, thereby separating CD14 positive cells. The CD14 positive cells containing human monocytes were suspended to 1 × 10⁶ cells/ml in a 10% FCS-containing RPMI 1640 medium. 50 ng/ml recombinant human GM-CSF (Kirin Brewery) and 100 ng/ml recombinant human IL-4 (R&D systems) were added to the medium, and then cultured in a 6-well culture plate (# 3046, Falcon). The cells cultured for 5 days were considered as human monocyte-derived immature DCs, and the cells cultured further for one more day after addition of 10 ng/ml LPS (SIGMA) were considered as human monocyte-derived mature DCs. The maturation of the human monocyte-derived DCs was confirmed by analyzing the up-regulation of HLA-DR and CD86 by flow cytometry.

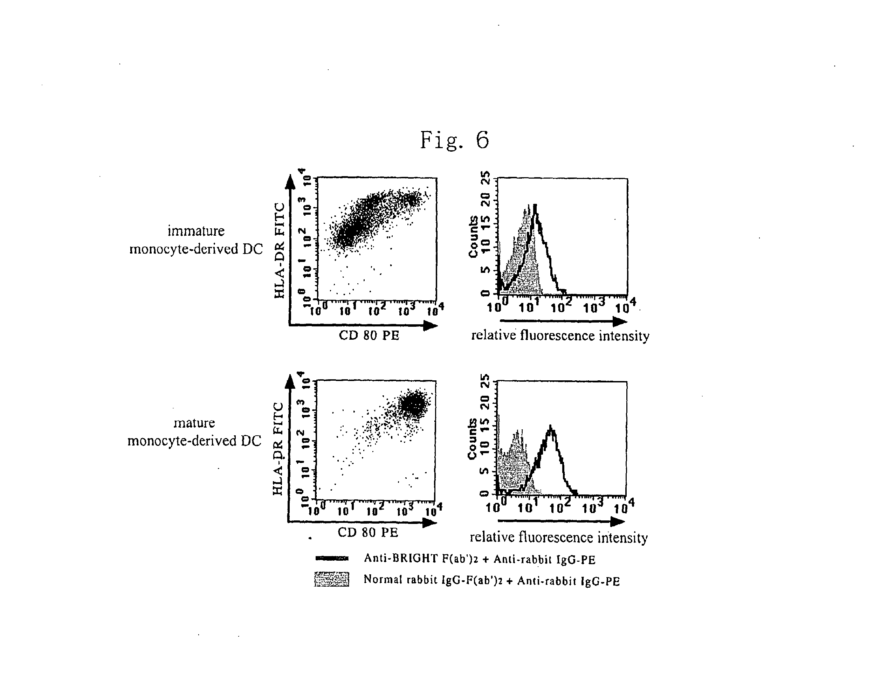

The mature or immature human monocyte-derived DCs were suspended in 1 ml of PBS-FCS-EDTA-NaN₃. 100 µg of human IgG was added to the suspension, and then incubated for 10 minutes on ice. Subsequently, 20 µg of anti-BRIGHT F(ab')₂ was added, and then incubated for 30 minutes on ice. 20 µg of normal rabbit IgG F(ab') was added to the cells of a control group. After the cells were washed with PBS-FCS-EDTA-NaN₃, PE-labeled anti-rabbit IgG antibody F(ab')₂ fragments (Southern Biotechnology Associates) were added, and then the mixture was incubated for 30 minutes on ice. The cells were washed with PBS-FCS-EDTA-NaN₃, and then the expression of the membrane molecule was analyzed by flow cytometer. The expression of BRIGHT was observed on the cell surfaces of immature DCs induced by human monocytes in the presence of recombinant human GM-CSF and recombinant human IL-4, and increased with maturation of DCs by LPS stimulation (see Fig. 6).

### Example 17 Analysis of BRIGHT counter receptor expressed on activated T cell

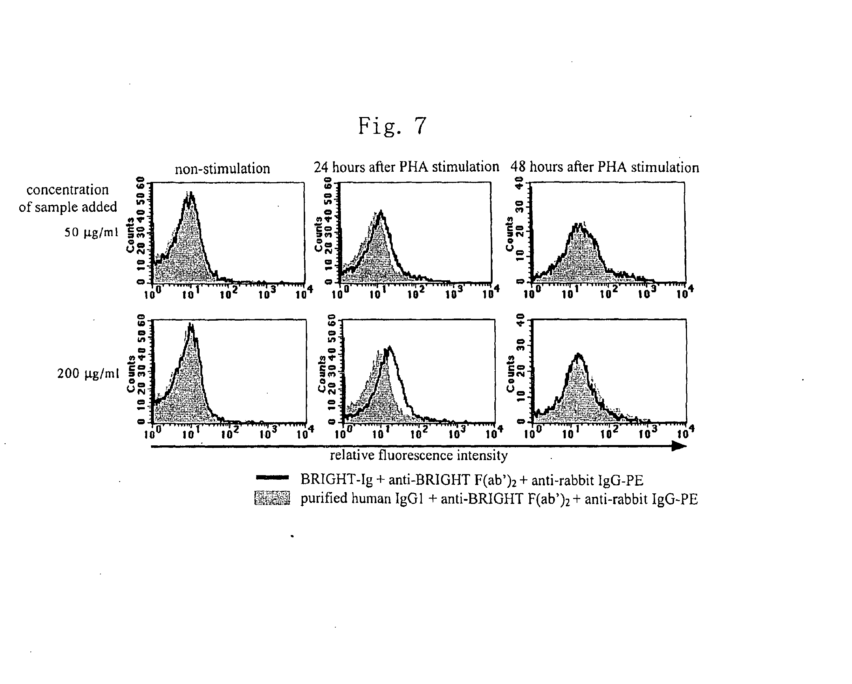

The amino acid sequence of BRIGHT has high homology with that of a costimulatory molecule belonging to known B7 family. Hence, also regarding function, BRIGHT is predicted to be a co-stimulatory molecule involved in regulation of T-cell activation via counter receptors on T cells. Actually, binding of soluble fusion proteins of human IgG1 Fc region and CD80 or CD86 with counter receptors CD28 expressed on T cells can be detected by flow cytometer. The expression of counter receptors on T cells was analyzed using BRIGHT-Ig described in Examples 10 and 11. Peripheral blood mononuclear cells collected from healthy individuals were separated by density gradient centrifugation using Ficoll-Paque. Using a Pan T Cell isolation kit (Miltenyi Biotec), CD3 positive T cells with purity of 99% or more were prepared by a negative selection method for removing cells other than T cells. In order to activate T cells, the purified CD3 positive T cells were suspended to 2 × 10⁶ cells/ml in RPMI 1640 medium with 10% FCS. 5 µg/ml PHA (phytohemagglutinin) (SEIKAGAKU CORPORATION) was added, and then cultured for 24 or 48 hours. T cells freshly isolated from peripheral blood and T cells activated with PHA were collected, washed with PBS-FCS, and then suspended in PBS (PBS-FCS-NaN₃) containing 3% FCS and 0.02% sodium azide. 200 µg/ml or 50 µg/ml BRIGHT-Ig was added to the suspension, and then incubated at 4°C for 1 hour. As a control group, 200 µg/ml or 50 µg/ml purified human IgG1 (SIGMA) was added, and then incubated similarly. The cells were washed with PBS-FCS-NaN₃, anti-BRIGHT F(ab')₂ was added, and then incubated at 4°C for 30 minutes. The cells were washed with PBS-FCS-NaN₃, PE-labeled anti-rabbit IgG antibody F(ab')₂ fragments (Southern Biotechnology Associates) were added, and then incubated for 30 minutes on ice. After the cells were washed, binding of BRIGHT-Ig to T cells was analyzed by flow cytometer. BRIGHT-Ig bound to activated T cells that had been cultured in the presence of PHA for 24 hours was detected, but did neither to CD3 positive T cells freshly isolated from peripheral blood and to T cells that had been cultured in the presence of PHA for 48 hours. These results suggested that BRIGHT counter receptors were present on activated T cells, and the expression was transiently increased in the early stage of T cell-activation. (See Fig. 7).

### Example 18 Suppressive effect of BRIGHT-Ig on T-cell proliferation

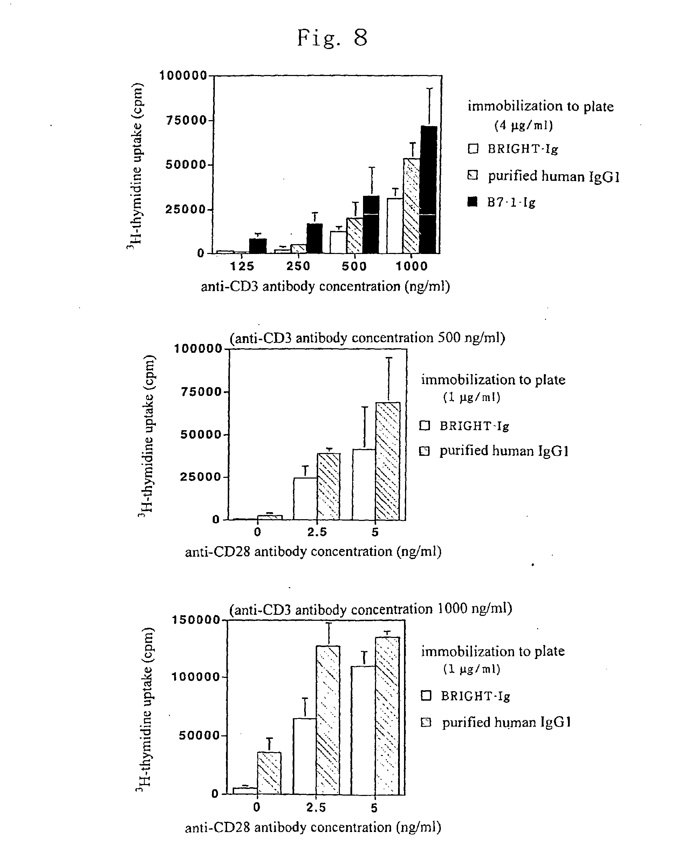

Whether BRIGHT is a co-stimulatory molecule involved in regulation of T-cell activation was examined by an in vitro T-cell proliferation experiment. T-cell proliferation requires simulation mediated by T-cell receptors (TCR), and in vivo, growth signal is transduced to T cells when TCR recognizes antigens presented by antigen-presenting cells, such as DC. TCR forms a complex with CD3 subunit group on T-cell membrane, and antigen stimulation from TCR is transduced via phosphorylation of CD3 subunits. It is known that when anti-CD3 antibodies stimulate T cells in vitro, growth signal similar to the stimulation through TCR can be transduced into the cells. This is used for in vitro T-cell proliferation experiments. Further, the effect of costimulatory molecules via CD28 molecules on T-cell proliferation can be examined by adding anti-CD28 antibodies, soluble CD80 molecules and soluble CD86 molecules at the same time to a culture system [J. Exp. Med., vol. 173, pp 721-730, 1991]. Thus, the effect of BRIGHT under stimulation of T-cell proliferation by anti-CD3 antibodies, and the effect of BRIGHT on T-cell proliferation under conditions wherein stimulation for activation by anti-CD28 antibodies were present in addition to anti-CD3 antibodies were examined. Specifically, anti-CD3 antibodies (BD Pharmingen) that had been diluted stepwise with a 50 mM sodium bicarbonate buffer (pH 9.0) were added 50 µl/ well in a flat bottom 96-well culture plate (# 1172, Falcon). The plate was incubated overnight at 4°C, so as to immobilize the antibodies on the culture plate. After the culture plate was washed, BRIGHT-Ig diluted with 50 mM sodium bicarbonate buffer (pH 9.0), and as a control group, purified human IgG1 or soluble human CD80 (R&D systems) were added, and the plate was incubated at 37°C for 4 hours for immobilization. When anti-CD28 antibodies were used, after anti-CD3 antibodies were immobilized, anti-CD28 antibodies diluted serially were immobilized, and then BRIGHT-Ig was immobilized. The culture plate with the antibodies and soluble molecules immobilized thereto was washed with PBS, and then used for a T-cell proliferation assay. T cells used herein were CD3 positive T cells with 99% or more purity that had been prepared by separating mononuclear cells from the peripheral blood of healthy individuals and then purifying the mononuclear cells using a Pan T Cell isolation kit (Miltenyi Biotec). T cells suspended in RPMI 1640 media with 10% FCS (GIBCO BRL) were added, 1 x 10⁵ cells/well/200 µl, to the above immobilized culture plate, and then cultured at 37°C for 3 days in the presence of 5% CO₂. ³H-thymidine (Amersham Pharmacia Biotech) was added, 0.25 µCi/well, to the culture plate on day 3 of culture, and then the plate was incubated at 37°C for 18 to 20 hours in the presence of 5% CO₂. Cells pulsed with ³H-thymidine were harvested onto Printed Filtermat A (Wallac) using Micro96 Harvester (SKATRON). After drying, the product was dipped well in Betap; Scint (Wallac), and then packaged. Then, activity was measured by measuring β ray dose using a 1205 BETAPLATE liquid scintillation counter (Wallac). The immobilized BRIGHT-Ig exhibited a suppressive effect on T cells proliferating under simulation by anti-CD3 antibodies. Moreover, under conditions wherein T-cell proliferation was induced by co-stimulation mediated by CD28 molecules, the immobilized BRIGHT-Ig also exhibited a suppressive effect on T-cell proliferation. (See Fig. 8)

### Example 19 Suppressive effect of BRIGHT on culturing of allogenic mixed lymphocytes

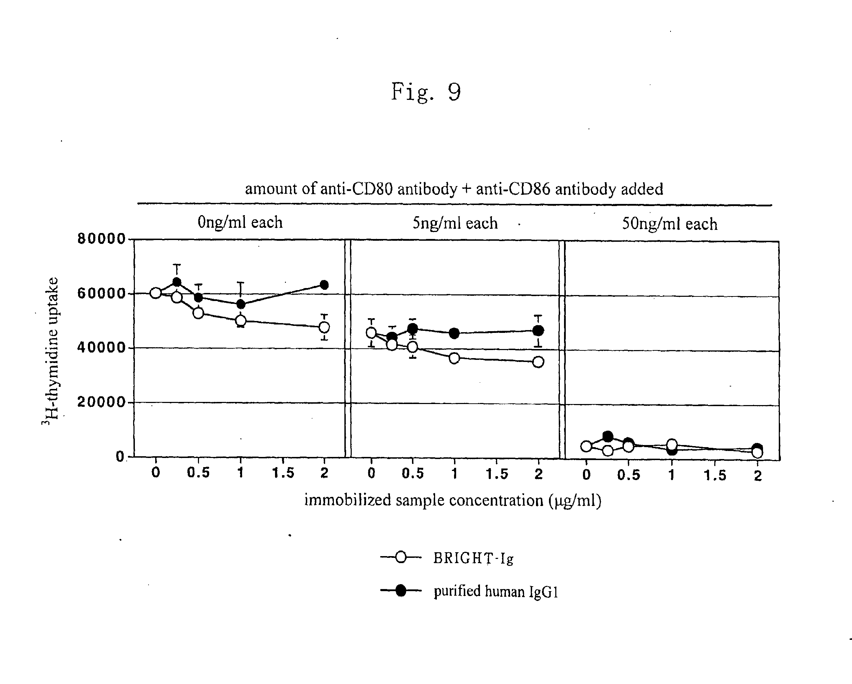

In allogenic transplantation with different major histocompatibility antigens (MHC), T cells are activated by recognizing non-self (histoincompatibility) MHC molecular complexes (alloantigen), so as to cause rejection. Human MHC is called HLA (human leukocyte antigen), and includes antigen class I to which HLA-A, B, and C belong, and antigen class II to which HLA-DP, DQ, and DR belong. Further, since each molecule has polymorphism, there may be several thousand combinations of human HLA, which makes the possibility of causing histoincompatibility between different individuals extremely high. Now, immunosuppressants, such as cyclosporin A and FK506, are used clinically to suppress rejection upon organ transplantation. However, the problems of these immunosuppressants are that they suppress immunoreaction non-specifically, so that they have strong side effects, and that they cannot induce immune tolerance to T cells, so that they have a weak effect on chronic rejection. On the other hand, regulation of signals mediated by costimulatory molecules has been shown by a transplantation experiment for a mouse model to be able to induce immune tolerance to T cells and suppress chronic rejection [Pro. Natl. Acad. Sci. USA, vol. 89, pp 11102 to 11105, 1992] [Science, vol. 257, pp 789 to792, 1992] [Nature, vol. 381, pp 434 to 438, 1996]. Thus, signal regulation mediated by BRIGHT is expected to suppress immune rejection and to induce immune tolerance. Culturing of allogenic mixed lymphocytes is an experiment to examine in vitro the proliferation of T cells that react with alloantigens when culturing mixed lymphocytes which differ in their corresponding histocompatibility antigens (hereinafter referred to as donor A and donor B, for convenience). Further, the reactivity of T cells against similar alloantigens can also be examined by carrying out mixed culturing of only monocyte-derived DC (mo-DC) induced in vitro from the peripheral blood of donor A, and only T cells separated from the peripheral blood of donor B. Thus, in the presence of immobilized BRIGHT-Ig, mixed culturing of allogenic DCs and T cells was performed, so that the effect of BRIGHT on T-cell alloantigen reactivity was examined. Specifically, BRIGHT-Ig that had been diluted stepwise with 50 mM sodium bicarbonate buffer (pH 9.0) or purified human IgG1 as a control group was apportioned 50 µl/well into a flat bottom 96-well culture plate (#1172, Falcon). The plate was allowed to stand at 37°C for 4 hours for immobilization. The culture plate with the soluble molecules immobilized thereto was washed with PBS, and then used for the mixed culturing experiment. According to the method described in Example 16, moriocyte-derived mature DCs that had been induced from the peripheral blood of donor A and activated with LPS were suspended to 1 × 10⁵ cells/ml in a 10% human plasma-containing RPMI 1640 medium (hereinafter referred to as RPMI-10% plasma). Further, according to the method described in Example 17, CD3 positive T cells with 99% or more purity that had been separated from the peripheral blood of donor B were suspended to 1 × 10⁶ cells/ml in RPMI-10% plasma. 100 µl each of the monocyte-derived DC suspension and the CD3 positive T cell suspension was mixed on a 96-well culture plate with BRIGHT-Ig immobilized thereto. As a control group, anti-CD80 antibodies and anti-CD86 antibodies were respectively added, before mixing, to the monocyte-derived DC suspensions to a concentration of 5 ng/ml or 50 ng/ml, and then the suspensions were allowed to stand for 30 minutes. Thus, the suspensions were mixed with the T-cell suspensions. The mixed cells were cultured at 37°C for 4 days in the presence of 5% CO₂, ³H-thymidine (Amersham Pharmacia Biotech) was added, 0.25 µCi/well, and then the plate was further allowed to stand at 37°C for 18 to 20 hours in the presence of 5% CO₂. ³H-thymidine incorporated by cells was collected onto Printed Filtermat (Wallac) using Micro96 Harvester (SKATRON). After drying, the product was dipped well in Betap; Scint (Wallac), and then packaged. Then, activity measurement was performed for β ray dose using a 1205 BETAPLATE liquid scintillation counter (Wallac). The immobilized BRIGHT-Ig suppressed in a concentration-dependent manner the proliferation of alloantigen reactive T cells. Further, the suppression activity mediated by BRIGHT was also observed under a condition wherein CD28-mediated costimulation had been partially suppressed by anti-CD80 antibodies and anti-CD86 antibodies, suggesting that BRIGHT acted on T cells independently from these costimulation pathways. (See Fig. 9)

### Example 20 Suppression by BRIGHT of expression of cytokine mRNA in allogeneic mixed leukocyte reaction

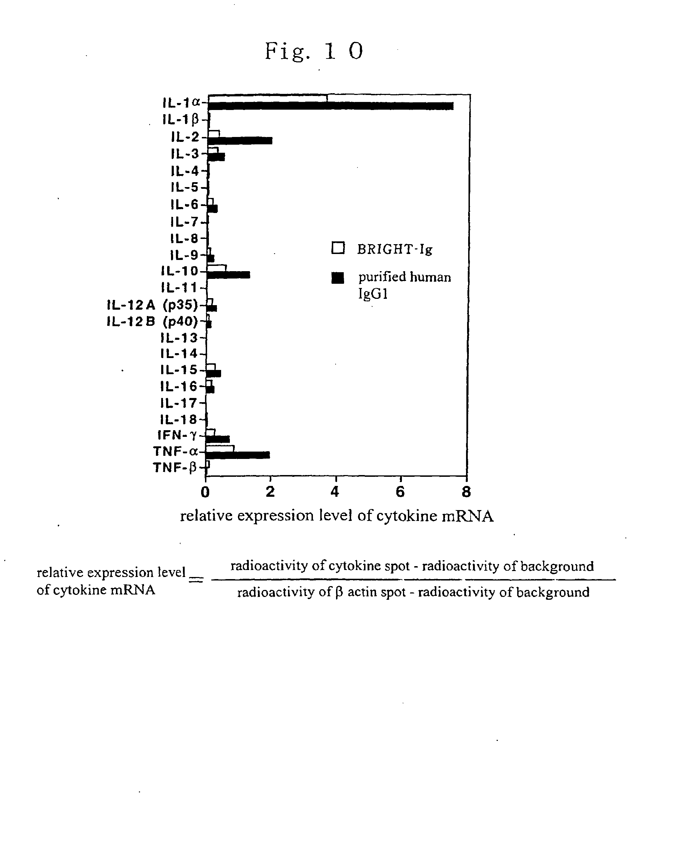

The results of Example 19 revealed that immobilized BRIGHT-Ig suppressed the proliferation of alloantigen-reactive T cells. Thus, the effect of BRIGHT-Ig on the expression of various cytokine mRNAs in allogeneic mixed leukocyte reaction was examined. Specifically, using a flat bottom 24-well culture plate (Falcon, #1147), BRIGHT-Ig or purified human IgG1 as a control was immobilized at a concentration of 4 µg/ml, similar to Example 19. According to the method described in Example 16, monocyte-derived immature DCs derived from donor A were suspended in RPMI-plasma. Further, according to the method described in Example 17, CD3 positive T cells with 99% or more purity derived from donor B were also suspended in RPMI-10% plasma. 0.9 × 10⁵ cells/well of monocyte-derived DCs and 1.2 × 10⁶ cells/well of CD3 positive T cells were mixed in the BRIGHT-Ig immobilized 24-well culture plate. After culturing at 37°C for 21 hours in the presence of 5% CO₂, the cells were collected from the plate, and then total RNA was prepared using ISOGEN-LS (NIPPON GENE). According to the protocols of GEArray KIT (Super Array), ³²P-labeled cDNA probe was prepared from 10 µg of total RNA by reverse transcription reaction using a human cytokine gene-specific primer set and Superscript II RNase H⁻ Reverse Transcriptase (GIBCO BRL) in the presence of [α-³²P]-dCTP (Amersham Pharmacia Biotech). Hybridization was performed with the membranes (Super Array) spotted with human cytokine gene-specific cDNA fragments using ³²P-labeled cDNA probes. The membranes were washed, and then radioactivity of the spots was analyzed using a FUJIX bio imaging analyzer BAS-2000II (FUJIFILM). The expression level of each cytokine gene was corrected based on the radioactivity of β-actin spotted on the same membrane. Specifically, the relative expression level of each cytokine mRNA was calculated according to the following correction formula. Cytokine mRNA relative expression level = (radioactivity of cytokine spot - radioactivity of background) ÷ (radioactivity of beta-actin spot - radioactivity of background). As shown in Figure 10, the immobilized BRIGHT-Ig significantly suppressed the expression of IL-1α, IL-2, IL-10, IFN-γ, and TNF-α mRNA in the allogeneic mixed leukocyte reaction.

### Example 21 Suppression by BRIGHT of cytokine secretion in allogenic mixed lymphocyte reaction

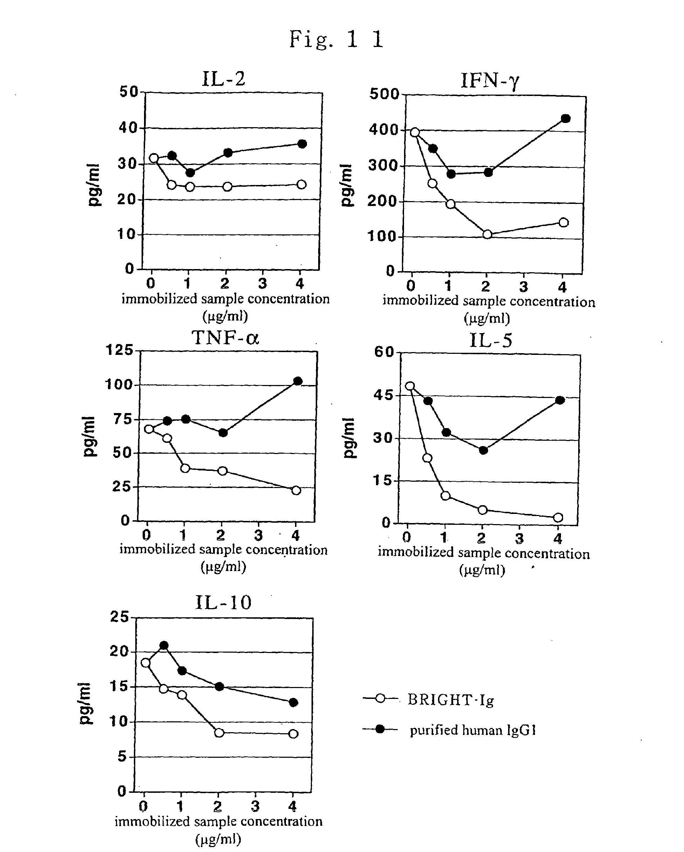

The effect of BRIGHT-Ig on secretion of various cytokines in allogenic mixed lymphocyte reaction was examined. Specifically, using a flat bottom 96-well culture plate (Falcon, #1172), BRIGHT-Ig diluted stepwise or purified human IgG1 as a control group were immobilized in a manner similar to the method described in Example 19. According to the method described in Example 16, monocyte-derived immature DCs that had been induced from the peripheral blood of donor A were suspended to 1.5 × 10⁵ cells/ml in RPMI-10% plasma. Further, according to the method described in Example 17, CD3 positive T cells with 99% or more purity that had been separated from the peripheral blood of donor B were suspended to 1.5 x 10⁶ cells/ml in RPMI-10% plasma. 100 µl each of the monocyte-derived DC suspension and the CD3 positive T cell suspension were mixed on the 96-well culture plate with BRIGHT-Ig immobilized thereto. After the mixed cells were cultured at 37°C for 3 days in the presence of 5% CO2, culture supernatant was collected. IL-2, IFN- γ, TNF- α, IL-5, and IL-10 secreted in the culture supernatant were measured by sandwich ELISA (R&D). Figure 11 shows the results. In the allogenic mixed lymphocyte reaction, secretion of IL-2, IFN-γ, TNF-α, IL-5, or IL-10 into the culture supernatant was suppressed depending on the concentration of BRIGHT-Ig immobilized. Thus, it was shown that immobilized BRIGHT-Ig also exhibited a suppressive effect on the production of the above cytokine proteins in the allogenic mixed lymphocyte reaction.

### Example 22 The effect of anti-BRIGHT F(ab')₂ on auto PBMC

Mononuclear cells of the peripheral blood of healthy individuals were prepared according to the method described in Example 14. Then the mononuclear cells were suspended to 1 x 10⁸ cells/ml in 2% human plasma-containing PBS (hereinafter, referred to as PBS-2% plasma). Anti-human CD14 antibody binding magnetic microbeads (Miltenyi Biotec) were added to the suspension, and then incubated for 30 minutes on ice. After washing with PBS-2% plasma, the cells were applied to a separation column LS+ (Miltenyi Biotec) within a magnetic field, thereby separating CD14 positive cells. The cells that had not been adsorbed to the column were collected and cultured. The fraction was referred to as CD14 negative PBMC. The CD14 negative PBMC was suspended at a concentration of 1.0 × 10⁶ cells/ml in a 5% human plasma-containing RPMI 1640 culture medium (hereinafter, referred to as RPMI-5% plasma). 200 µl of the suspension was added to each well of a flat bottom 96-well culture plate (#3072, Falcon), and then anti-BRIGHT F(ab')₂ or normal rabbit IgG F(ab')₂ in an amount as described below was added. The cells were then cultured at 37°C for 3 days or 4 days in the presence of 5% CO₂. 100 µCi/ml ³H-thymidine (Amersham Pharmacia Biotech) was added, 10 µl/well, to the culture plate at 3 or 4 days after culturing. The plate was further incubated at 37°C for 14 hours in the presence of 5% CO₂. Up-take of ³H-thymidine (Amersham Pharmacia Biotech) was measured as described in Example 18. 7 groups were set: (i) anti-BRIGHT F(ab')₂ (1.72 mg/ml) was added at 10 µl/well, (ii) anti-BRIGHT F(ab')₂ (1.72 mg/ml) was added at 3 µl/well, and RPMI-5% plasma was added at 7 µl/well, (iii) anti-BRIGHT F(ab')₂ (1.72 mg/ml) was added at 1 µl/well and RPMI-5% plasma was added at 9 µl/well, (iv) normal rabbit IgG F(ab')₂ (1.48 mg/ml) was added at 10 µl/well, (v) normal rabbit IgG F(ab')₂ (1.48 mg/ml) was added at 3 µl/well, and RPMI-5% plasma was added at 7 µl/well, (vi) normal rabbit IgG F(ab')₂ (1.48 mg/ml) was added at 1 µl/well, and RPMI-5% plasma was added at 9 µl/well, and (vii) RPMI-5% plasma was added at 10 µl/well. The same conditions were applied for every 3 wells per group. Values measured by the liquid scintillation counter were averaged for every 3 wells under the same conditions, and the average was used as the experimental value of each group.

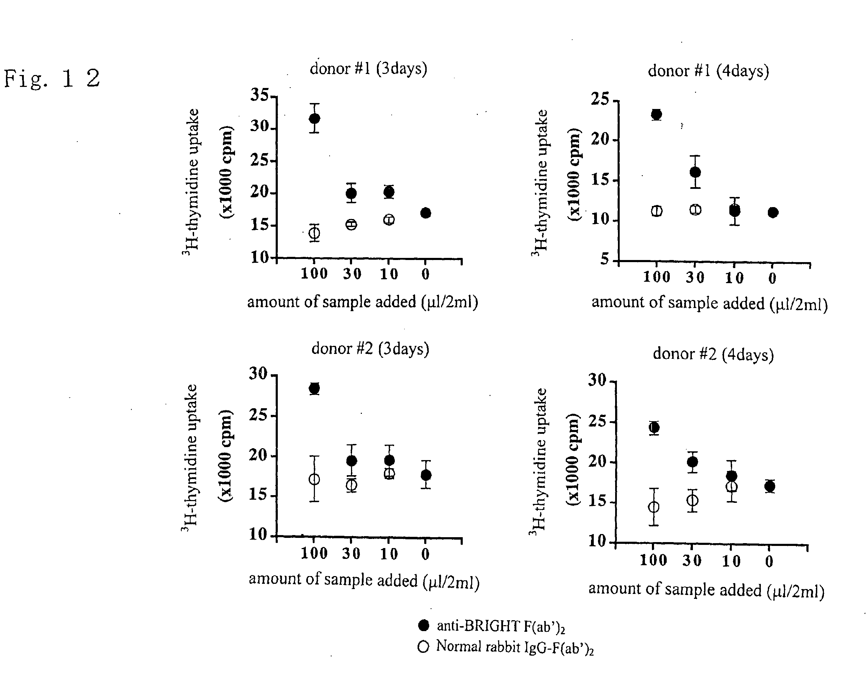

As a result, in the cultured cells derived from multiple individuals, the count of anti-BRIGHT F(ab')₂-added group (i) or (ii) significantly increased compared to that of the control group, normal rabbit IgG F(ab')₂-added group (iv) or (v). The increase in the count showed a tendency of being dependent on the amount of anti-BRIGHT F(ab')₂ added. Therefore, it was shown that the cell proliferation activity in the human peripheral blood CD14 negative PBMC was enhanced by anti-BRIGHT F(ab')₂. (See Fig. 12).

### Example 23 Flow cytometer analysis of peripheral blood mononuclear cells of healthy individuals after effect of anti-BRIGHT F(ab')₂

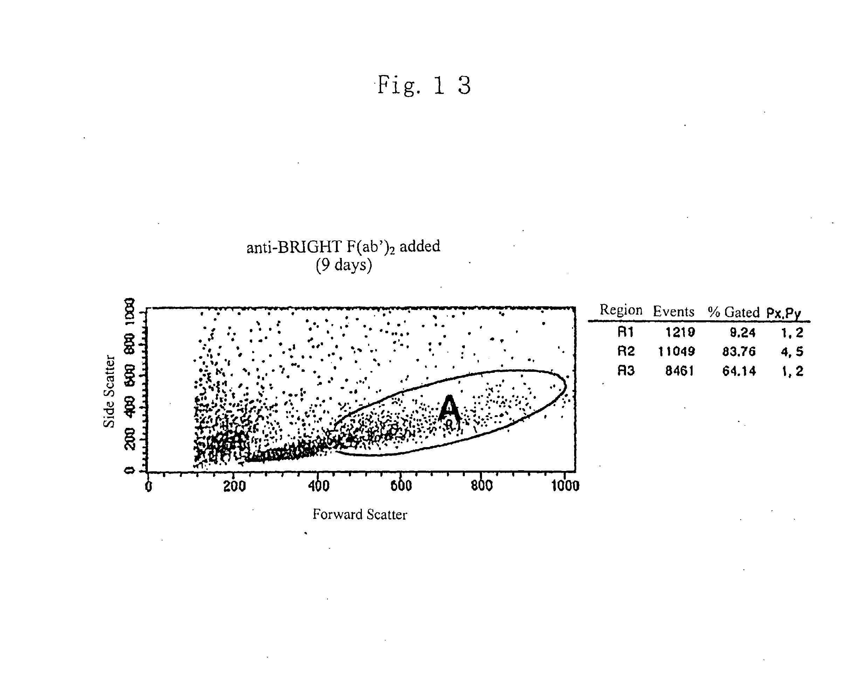

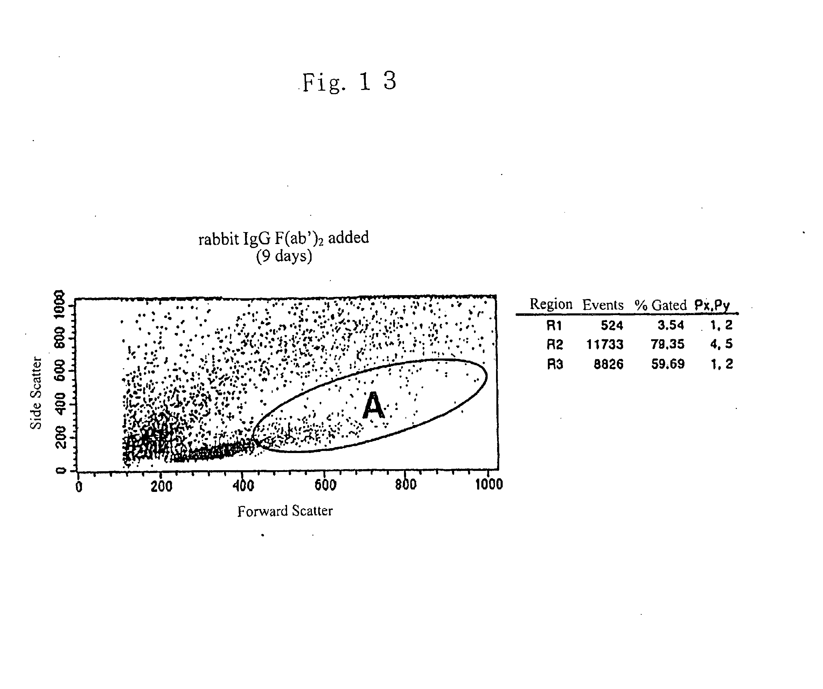

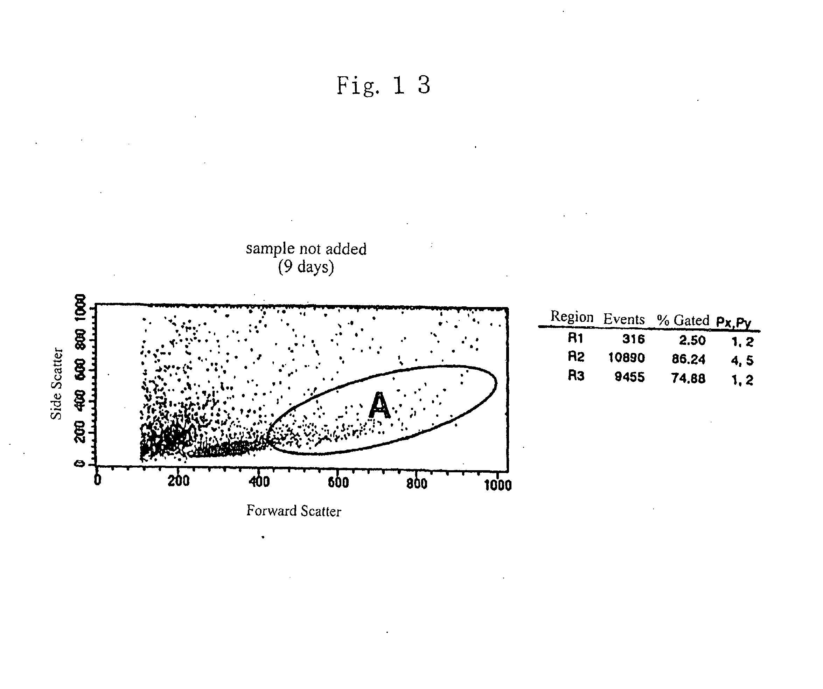

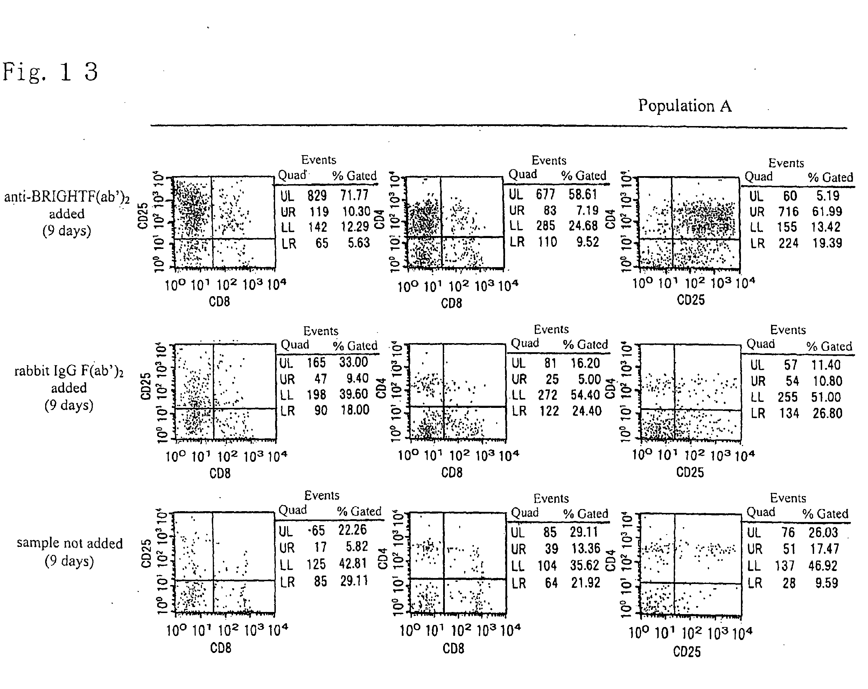

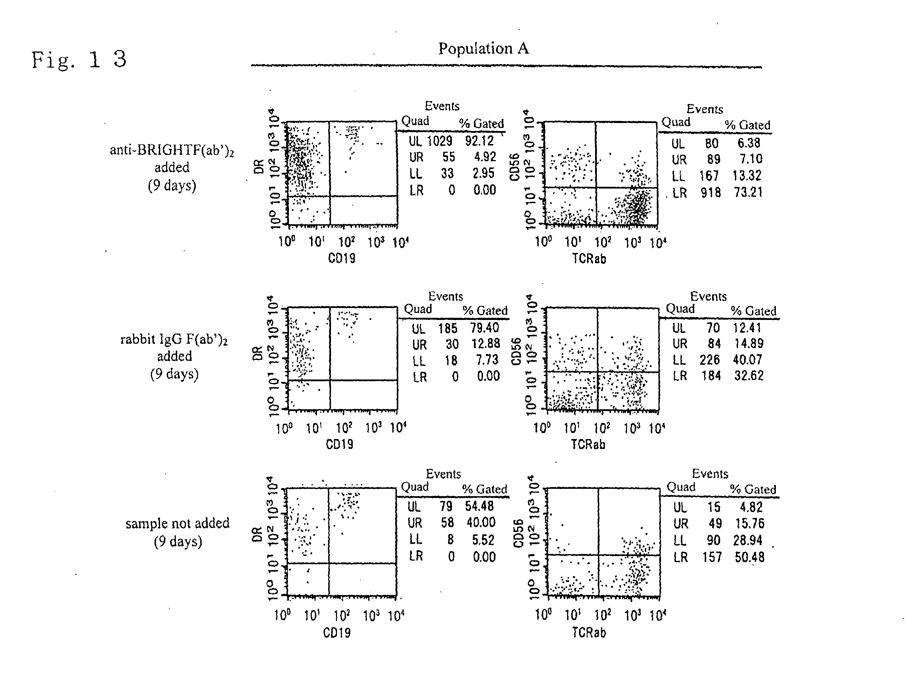

The cells were suspended at a concentration of 1.0 x 10⁶ cells/ml in RPMI-5% plasma. 1 ml of the cell suspension was inoculated in each well of a flat bottom 48-well culture plate (# 3078, Falcon). After anti-BRIGHT F(ab')₂ or normal rabbit IgG F(ab')₂ was added in an amount described below, culturing was performed at 37°C for 9 days in the presence of 5% CO₂. The cells were stained using the following antibodies: anti-CD4-APC labeled (BD Pharmingen), anti-CD8-FITC labeled (BD Pharmingen), anti-CD25-PE labeled (BD Pharmingen), anti-HLA-DR-FITC labeled (BD Pharmingen), anti-TCRαβ-PE labeled (Beckman Coultar), and anti-CD56-APC labeled (Beckman Coultar) antibodies. Then analysis was performed by FACScalibur (Becton Dickinson). 3 groups were set as follows: (i) anti-BRIGHT F(ab')₂ (1.72 mg/ml) was added at 50 µl/well (ii) normal rabbit IgG F(ab')₂ (1.48 mg/ml) was added at 50 µl/well (iii) RPMI-5% plasma was added at 50 µl/well. As a result, in (i), cells that increase in size and take a form containing granules (Large granular lymphocyte; LGL) were observed in greater number compared to (ii) and (iii). When the surface antigens of the cells were analyzed, TCRαβ positive cells were 82%, HLA-DR positive cells were 97%, CD4 positive cells were 68%, and CD25 positive cells were 82%. These results revealed that LGL-like cells increased by anti-BRIGHT F(ab')₂ and most of these cells were activated CD4 positive T cells. (See Fig. 13).

### Example 24 Effect of anti-BRIGHT F(ab')₂ in experiment of auto mixed lymphocvte reaction (auto MLR) activated by super antigen

Monocyte-derived DCs were prepared according to the method described in Example 16. The monocytes were separated from the peripheral blood of the same healthy individuals, and then CD3 positive T cells with 99% or more purity were prepared using a Pan T Cell isolation kit (Miltenyi Biotec). The monocyte-derived DCs and CD3 positive T cells were mixed, and then auto MLR was performed using a flat bottom 96-well culture plate (#3072, Falcon). Staphylococcus Enterotoxins (SEE), a type of super antigen, was added as a stimulation antigen directly to a reaction system at a final concentration of 0.3 ng/ml or 0.1 ng/ml or 0.03 ng/ml. T/DC cell ratios employed herein were 10, 20 and 40, and T cell count employed herein was 1.5 x 10⁵ cells/200µl/well. Anti-BRIGHT F(ab')₂ or normal rabbit IgG F(ab')₂ were added at a final concentration of 10, 2, 0.4, and 0 µg/ml to DCs, followed by reaction for 30 minutes under ice cooling. After addition of SEE in an amount as described above, reaction was further continued for 30 minutes under ice cooling. Next, T cells were added to the solution, and then cultured at 37°C for 2 days in the presence of 5% CO₂. 100 µCi/ml ³H-thymidine (Amersham Pharmacia Biotech) was added, 10 µl/well, to the culture plate at 3 or 4 days after culturing. The plate was allowed to stand at 37°C for 14 hours in the presence of 5% CO₂. ³H-thymidine incorporated by cells was collected onto Printed Filtermat A (Wallac) using Micro96 Harvester (SKATRON). After drying, the product was dipped well in Betap; Scint (Wallac), and then packaged. Activity measurement was then performed for beta ray dose using 1205 BETAPLATE liquid scintillation counter (Wallac).

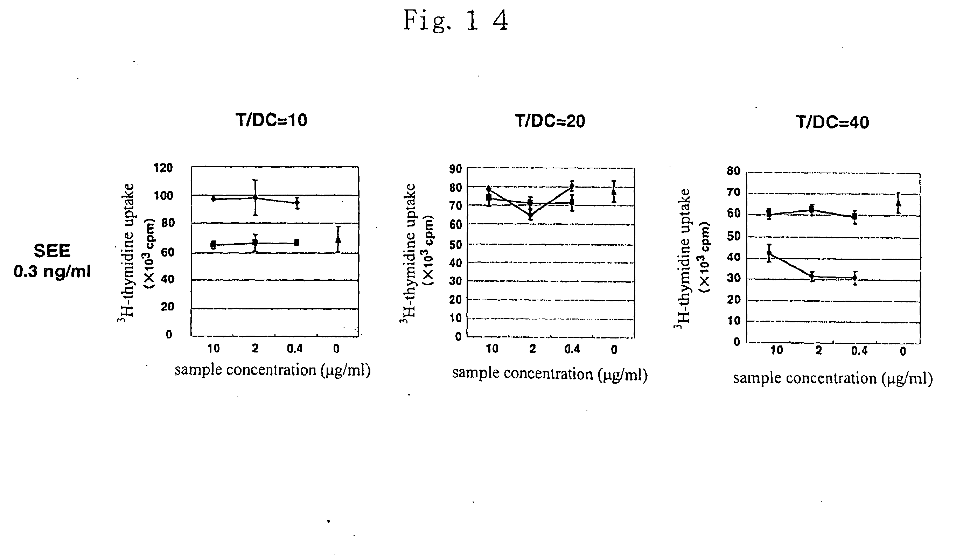

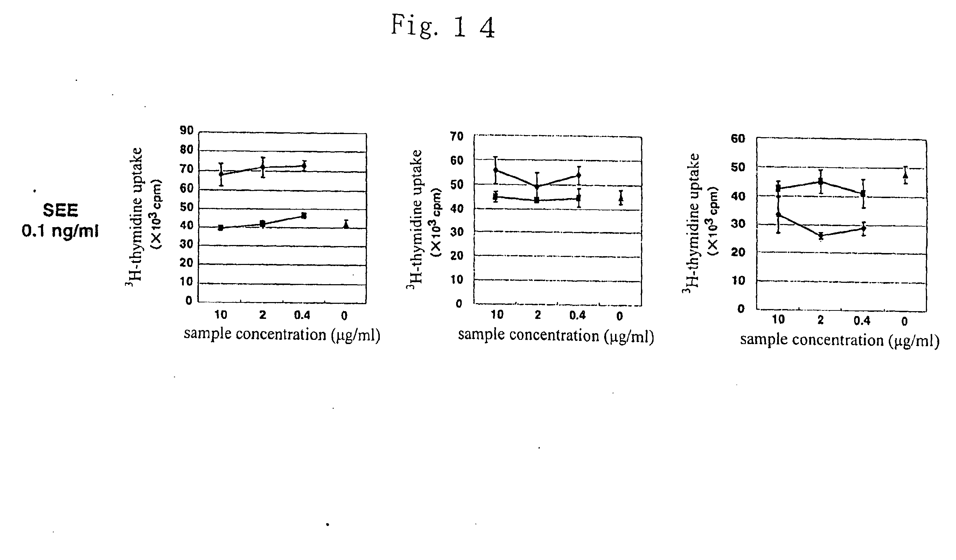

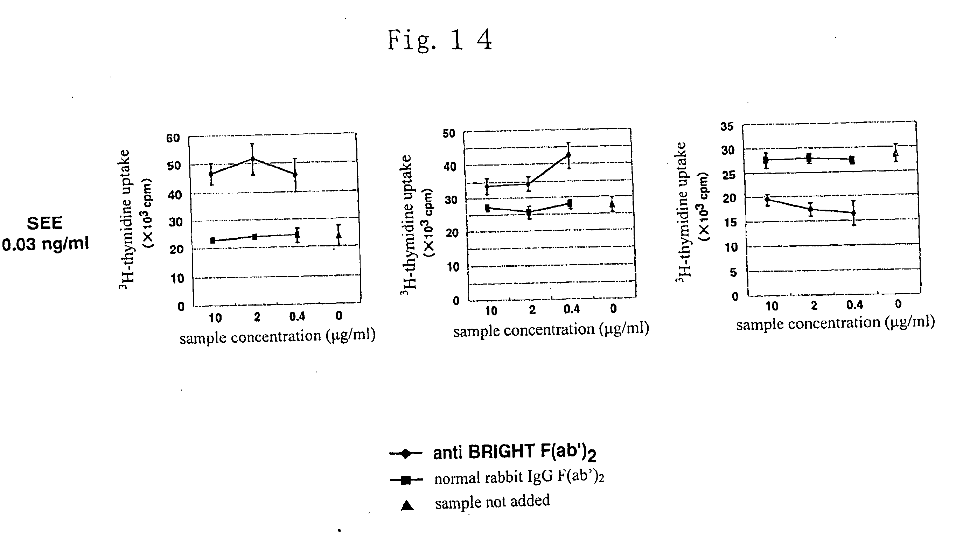

As a result, in the cultured cell derived from multiple individuals, the count of anti-BRIGHT F(ab')₂-added group showed a value different from that of the control group, the normal rabbit IgG F(ab')₂-added group. Thus, it was shown in the experiment of mixed reaction of autolymphocytes that anti-BRIGHT F(ab')₂ exhibited the effect on T cells mainly in a T/DC cell ratio-dependent manner (See Fig. 14).

### Example 25 Effect of anti-BRIGHT F(ab')₂ on IgM production from B cells by DC

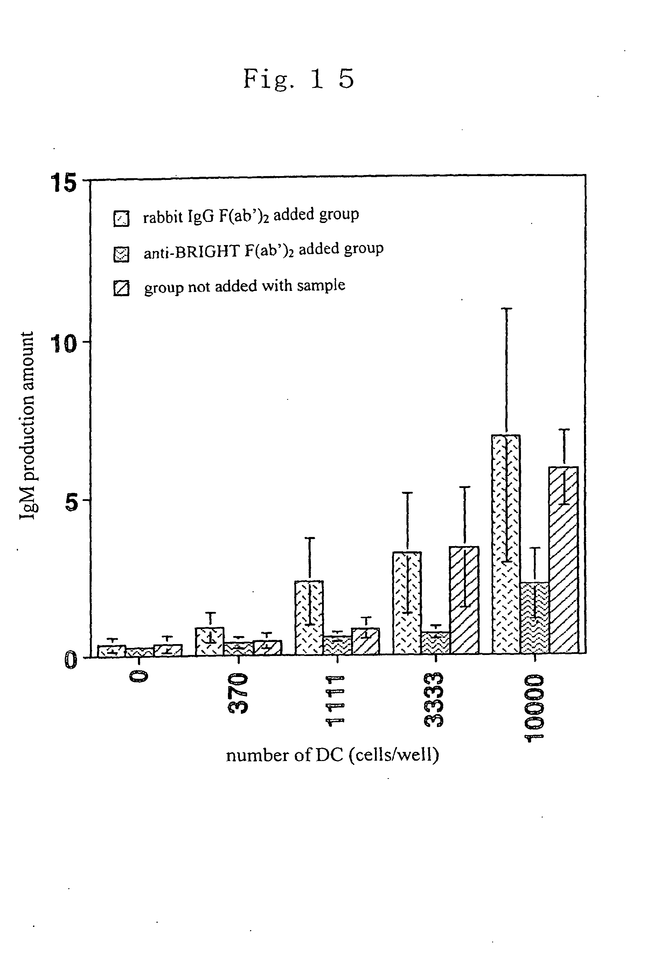

Involvement of DC in the growth of B cells has been reported [J. Exp. Med., vol. 185, pp 941-952, 1997]. Particularly for in vitro, it is known that direct cell-cell interaction between DC and B cells is involved in IgM production upon the addition of IL-2. Mononuclear cells separated from the peripheral blood of healthy individuals by density gradient centrifugation using Lymphoprep (Nycomed) were suspended to 1 × 10⁸ cells/ml in PBS containing 2% bovine serum (PBS-2% FCS). Anti-human CD19 antibody binding magnetic microbeads (Miltenyi Biotec) were added to the suspension, and then incubated for 30 minutes on ice. After washing with PBS-FCS, the cells were suspended in PBS containing 5% BSA and 10 mM EDTA (PBS-BSA-EDTA), and then applied to a separation column LS+ (Miltenyi Biotec) in a magnetic field, thereby separating CD19 positive cells. Further, according to the method described in Example 16, monocyte-derived immature DCs were prepared. 50 ng/ml recombinant human TNF-α (Genzyme Techne) was added to the cells, and then the cells were cultured at 37°C for 3 days in the presence of 5% CO₂, so as to induce the maturation of DCs. 2 µg/well/50 µl of anti-BRIGHT F(ab')₂ or normal rabbit IgG F(ab')₂ was added to a flat bottom 96-well culture plate (# 3072, Falcon). Then, TNF-α matured DCs suspended in 10% FCS containing IMDM medium (GIBCO BRL) were added at 1 × 10⁴, 3.3 × 10³, 1.1 × 10³, 3.7 × 10², and 0 cells/well/50 µl, and incubated at 37°C for 30 minutes in the presence of 5% CO₂. CD40L transfected L cells (2.5 × 10³ cells/well/50 µl), B cells (2 × 10⁴ cells/well/50 µl) (the growth of these cells had been stopped by X-ray irradiation at 7500 rad), and 50 unit/ml recombinant human IL-2 (Genzyme) were added, and then cultured at 37 °C for 15 days in the presence of 5% CO₂. IgM levels in the supernatants were measured by the Sandwich ELISA method using rabbit anti-human IgM (DAKO) as capture antibodies and peroxidase-conjugated F(ab')₂ fragment of rabbit anti-human IgM (DAKO) as detection antibodies. The results revealed that anti-BRIGHT F(ab')₂ showed a tendency to suppress IgM production compared to the control group with normal rabbit IgG F(ab')₂. (See Fig. 15)

### Sequence Listing Free Text

Explanation of SEQ ID NO: 5 - artificial sequence: sense primer.
Explanation of SEQ ID NO: 6 - artificial sequence: antisense primer.
Explanation of SEQ ID NO: 7 - artificial sequence: fusion protein of BRIGHT extracellular domain (29-465) and human IgG1 Fc domain

### Industrial Applicability

The present invention enables not only selective separation of DC with high purity from other blood cells, but also separation of mature DC from immature DC, so that it enables to supply the cells to DC therapy. Separated DC is expected to be used as, for example, a cancer vaccine by re-infusing the separated DC to a patient after antigens are pulsed. Moreover, it is also expected that immune response can be regulated by enhancing or suppressing interaction between DC and T cells using antibodies against the membrane molecule or soluble molecules of the membrane molecule.

All publications, patents and patent applications cited herein are inserted herein by reference in their entirety.

## Claims

1. An isolated human dendritic cell membrane molecule, which has an amino acid sequence represented by SEQ ID NO: 1, or a variant thereof, which has an amino acid sequence derived from the amino acid sequence by deletion, substitution, insertion and/or addition of one or more amino acid residues and is capable of regulating immune response.

2. The variant of claim 1, which contains an amino acid sequence from position 29 to 465 of SEQ ID NO: 1 corresponding to an extracellular domain.

3. The variant of claim 2, which is a fusion protein of the protein having the amino acid sequence of position 29 to 465 of SEQ ID NO: 1 and another protein.

4. The variant of claim 3, wherein the other protein is derived from a human.

5. The variant of claim 3, which is a fusion protein of the protein having an amino acid sequence of position 29 to 465 of SEQ ID NO: 1 and a human IgGlFc domain.

6. The human dendritic cell membrane molecule or the variant thereof of any one of claims 1 to 5, which has an effect to suppress proliferation and activation of T cells.

7. A DNA which encodes the human dendritic cell membrane molecule or a variant thereof of any one of claims 1 to 6, or a complementary DNA thereof.

8. The DNA or the complementary DNA thereof of claim 7, which contains a nucleotide sequence encoding an amino acid sequence of SEQ ID: 1 or a partial sequence thereof.

9. The DNA or the complementary DNA thereof of claim 7, which contains a nucleotide sequence encoding an amino acid sequence of position 29 to 465 of SEQ ID NO: 1.

10. The DNA or the complementary DNA thereof of claim 7, which has a nucleotide sequence selected from the group consisting of SEQ ID NO: 2, SEQ ID NO: 7 and SEQ ID NO: 8.

11. An antibody or a fragment thereof, which specifically and immunologically binds to the human dendritic cell membrane molecule or a variant thereof of any one of claims 1 to 6 or fragments thereof.

12. The antibody or the fragment thereof of claim 11, which is **characterized by** recognizing an extracellular region of the human dendritic cell membrane molecule.

13. The antibody or the fragment thereof of claim 11 or 12, which is a polyclonal antibody, peptide antibody or monoclonal antibody.

14. The antibody or the fragment thereof of any one of claims 11 to 13, wherein the fragment is F(ab')₂.

15. The antibody or the fragment thereof of any one of claims 11 to 14, which has an effect of enhancing proliferation and activation of T cells.

16. The antibody or the fragment thereof of any one of claims 11 to 14, which has a characteristic of enhancing or suppressing proliferation of T cells according to the cell ratio of T cells and dendritic cells.

17. The antibody or the fragment thereof of any one of claims 11 to 14, which has activity to suppress IgM production by B cells.

18. A method for separating human-derived or other animals-derived mature dendritic cells using the antibody or the fragment thereof of any one of claims 11 to 17.

19. A method for detecting mature dendritic cells using the antibody or the fragment thereof of any one of claims 11 to 17.
